# EUROPEAN PATENT SPECIFICATION

(11) **EP 3 104 852 B1**
(45) Date of publication and mention of the grant of the patent: **14.08.2024**
(21) Application number: 15749364.4
(22) Date of filing: 11.02.2015
(51) Int. Cl.: A61K 31/225, A61K 31/19, C12N 5/02, A61K 31/194, A61K 31/198, A61P 35/00, A61P 25/00, A61P 19/02, A61P 7/00, A61P 3/00

(54) **COMPOSITIONS AND METHODS FOR TREATING AGING AND AGE-RELATED DISEASES AND SYMPTOMS**
ZUSAMMENSETZUNGEN UND VERFAHREN ZUR BEHANDLUNG VON ALTERS- UND ALTERSBEDINGTEN ERKRANKUNGEN UND SYMPTOMEN
COMPOSITIONS ET PROCÉDÉS DE TRAITEMENT DES MALADIES ET SYMPTÔMES DU VIEILLISSEMENT ET LIÉS À L'ÂGE

(30) Priority: 12.02.2014 US 201461939092 P; 19.03.2014 US 201461955463 P
(43) Date of publication of application: 21.12.2016
(73) Proprietor: THE REGENTS OF THE UNIVERSITY OF CALIFORNIA, Oakland, CA 94607-5200 (US)
(72) Inventor: HUANG, Jing, Los Angeles, California 90095-1406 (US); CHIN, Randall, Los Angeles, California 90095-1406 (US); DIEP, Simon, Los Angeles, California 90095-1406 (US); PAI, Melody Y., Los Angeles, California 90095-1406 (US); LOMENICK, Brett E., Los Angeles, California 90095-1406 (US); FU, Xudong, Los Angeles, California 90095-1406 (US); REUE, Karen, Los Angeles, California 90095-1406 (US); VERGNES, Laurent, Los Angeles, California 90095-1406 (US); JUNG, Michael E., Los Angeles, California 90095-1406 (US); DENG, Gang, Los Angeles, California 90095-1406 (US); HWANG, Heejun, Los Angeles, California 90095-1406 (US); JIANG, Meisheng, Los Angeles, California 90095-1406 (US)
(74) Representative: Bartle Read
(86) International application number: PCT/US2015/015304
(87) International publication number: WO 2015/123229

(56) References cited:
- WO-A1-2006/016143
- WO-A1-2009/005464
- WO-A2-2011/163319
- WO-A2-2015/049365
- US-A1- 2006 166 975
- US-A1- 2008 279 786
- US-A1- 2010 048 697
- ADRIAN P. HARRISON: "Biological effects of 2-oxoglutarate with particular emphasis on the regulation of protein, mineral and lipid absorption/metabolism, muscle performance, kidney function, bone formation and cancerogenesis, all viewed from a healthy ageing perspective state of the art--review article.", 1 August 2008 (2008-08-01), Journal of physiology and pharmacology : an official journal of the Polish Physiological Society, pages 91 - 106, XP055386333, Retrieved from the Internet <URL:https://www.researchgate.net/profile/Adrian_Harrison/publication/51433812_Biological_effects_of_2-oxoglutarate_with_particular_emphasis_on_the_regulation_of_protein_mineral_and_lipid_absorptionmetabolism_muscle_performance_kidney_function_bone_formation_and_cancerogenesis_a/links/55ba062408aed621de0> [retrieved on 20170629]
- T. NIEMIEC: "Alpha-ketoglutarate stabilizes redox homeostasis and improves arterial elasticity in aged mice", 1 February 2011 (2011-02-01), Journal of physiology and pharmacology : an official journal of the Polish Physiological Society, pages 37 - 43, XP055386339, Retrieved from the Internet <URL:http://www.jpp.krakow.pl/journal/archive/02_11/pdf/37_02_11_article.pdf> [retrieved on 20170629]
- EUI DONG SON ET AL: "Alpha-ketoglutarate stimulates procollagen production in cultured human dermal fibroblasts, and decreases UVB-induced wrinkle formation following topical application on the dorsal skin of hairless mice.", BIOLOGICAL & PHARMACEUTICAL BULLETIN (OF JAPAN), vol. 30, no. 8, 1 August 2007 (2007-08-01), JP, pages 1395 - 1399, XP055385759, ISSN: 0918-6158, DOI: 10.1248/bpb.30.1395
- DAVID S. WILLIAMS ET AL: "Oxaloacetate supplementation increases lifespan in Caenorhabditis elegans through an AMPK/FOXO-dependent pathway", AGING CELL, vol. 8, no. 6, 1 December 2009 (2009-12-01), GB, pages 765 - 768, XP055385766, ISSN: 1474-9718, DOI: 10.1111/j.1474-9726.2009.00527.x
- MICHAEL E. JUNG ET AL: "Synthesis of the 1-Monoester of 2-Ketoalkanedioic Acids, for Example, Octyl [alpha]-Ketoglutarate", THE JOURNAL OF ORGANIC CHEMISTRY, vol. 77, no. 23, 7 December 2012 (2012-12-07), pages 11002 - 11005, XP055385757, ISSN: 0022-3263, DOI: 10.1021/jo302308q
- E. D. MACKENZIE ET AL: "Cell-Permeating alpha-Ketoglutarate Derivatives Alleviate Pseudohypoxia in Succinate Dehydrogenase-Deficient Cells", MOLECULAR AND CELLULAR BIOLOGY., vol. 27, no. 9, 1 May 2007 (2007-05-01), US, pages 3282 - 3289, XP055359584, ISSN: 0270-7306, DOI: 10.1128/MCB.01927-06
- MAGI ET AL.: "Glutamate-Induced ATP Synthesis: Relationship between Plasma Membrane Na1/Ca21 Exchanger and Excitatory Amino Acid Transporters in Brain and Heart Cell Models", MOLECULAR PHARMACOLOGY, vol. 84, no. 4, 1 October 2013 (2013-10-01), pages 603 - 614, XP055219793
- CARDACI ET AL.: "TCA Cycle Defects and Cancer: When Metabolism Tunes Redox State.", INT J CELL BIOL., vol. 2012, no. 7363, 2012, pages 1 - 9, XP055219946
- MESSRIPOUR ET AL.: "Effects of Vitamin B6 on the Brain Glutamate Pyrovate Transaminase and Glutamate Oxaloacetate Transaminase in Young and Old Rats.", AMERICAN JOURNAL OF MEDICINE AND MEDICAL SCIENCES, vol. 2, no. 1, 2012, pages 33 - 35, XP055219951
- ZHAO ET AL.: "Inhibition of the ecto-beta subunit of FIF0-ATPase inhibits proliferation and induces apoptosis in acute myeloid leukemia cell lines.", JOURNAL OF EXPERIMENTAL & CLINICAL CANCER RESEARCH, vol. 31, no. 1, 9 November 2012 (2012-11-09), pages 1 - 9, XP021122942
- CHIN ET AL.: "The metabolite alpha-ketoglutarate extends lifespan by inhibiting the ATP synthase and TOR.", NATURE, vol. 510, no. 7505, June 2014 (2014-06-01), pages 397 - 401, XP055219964

## Description

### BACKGROUND OF THE INVENTION

### 1. FIELD OF THE INVENTION

The present invention relates to treating aging and age-related diseases.

### 2. DESCRIPTION OF THE RELATED ART

Metabolism and aging are intimately linked. Compared to ad libitum feeding, dietary restriction (DR) or calorie restriction (CR) consistently extends lifespan and delays age-related diseases in evolutionarily diverse organisms. Similar conditions of nutrient limitation and genetic or pharmacological perturbations of nutrient or energy metabolism also have longevity benefits. Several compounds that modulate aging with largely undefined molecular mechanisms have been identified.

WO 2009/005464 discloses the use of alpha-ketoglutarate, amides, and salts and mixtures thereof for the manufacture of a pharmaceutical preparation or a food or feed supplement for the in vivo therapeutic improvement of blood vessel elasticity.

WO2015/049365 discloses inducing autophagy in a cell comprising contacting the cell with at least one acetyl CoA (AcCoA) depleting agent.

However, a need still exists for treatments for age-related diseases including cancer, diabetes, and cardiovascular disease, and extending the lifespans of subjects.

### SUMMARY OF THE INVENTION

The present invention is set out in the appended claims.

In some embodiments, the one or more compounds are administered in an effective amount or a therapeutically effective amount. In some embodiments, the effective amount or the therapeutically effective amount of the one or more compounds is administered as several doses over a given period of time, e.g., a daily dose for a week or more. In some embodiments, the amount of the one or more compounds administered to the subject increases the a-ketoglutarate levels in the subject by about 30-60%, preferably about 45-55%, more preferably about 50%. In some embodiments, the compound is administered as a daily dose of about 0.25-2, preferably about 0.5-2, more preferably about 1-2, most preferably about 2, grams per kilogram weight of the subject per day. In some embodiments, the subject is an animal, which may or may not be an animal model of aging or an age-related disease. In some embodiments, the subject is a nematode, a rodent, or a non-human primate. In some embodiments, the subject is a human. In some embodiments, the ATP synthase is mammalian ATP synthase, human ATP synthase, mammalian mitochondrial ATP synthase, or human mitochondrial ATP synthase.

In some embodiments, the present invention provides a glutarate compound or a glutamate compound for use in inhibiting, reducing, slowing, or preventing the aging of a subject; treating, inhibiting, reducing, or preventing an age-related disease in the subject; and/or increasing the lifespan of the subject. In some embodiments, the compound is a glutarate compound or a glutamate compound. In some embodiments, the compound is a glutarate compound and a glutamate compound. In some embodiments, the compound is an a-KG compound. In some embodiments, the compound is a 2-HG compound. In some embodiments, the compound is an a-KG compound and a 2-HG compound. In some embodiments, the compound is provided in an effective amount or a therapeutically effective amount. In some embodiments, the medicament is provided as divided doses. In some embodiments, the effective amount or the therapeutically effective amount is provided as divided doses. In some embodiments, the subject is an animal, which may or may not be an animal model of aging or an age- related disease. In some embodiments, the subject is a nematode, a rodent, or a non- human primate. In some embodiments, the subject is a human. In some embodiments, the ATP synthase is mammalian ATP synthase, human ATP synthase, mammalian mitochondrial ATP synthase, or human mitochondrial ATP synthase.

Both the foregoing general description and the following detailed description are exemplary and explanatory only and are intended to provide further explanation of the invention as claimed. The accompanying drawings are included to provide a further understanding of the invention and are incorporated in and constitute part of this specification, illustrate several embodiments of the invention, and together with the description serve to explain the principles of the invention.

### DESCRIPTION OF THE DRAWINGS

This invention is further understood by reference to the drawings wherein:
Figure 1, Panels a-f, shows that a-KG extends the adult lifespan of C. *elegans.*
Figure 2, Panels a-i, shows that a-KG binds and inhibits ATP synthase.
Figure 3, Panels a-g, shows that a-KG longevity is mediated through ATP synthase and the DR/TOR axis.
Figure 4, Panels a-e, shows that inhibition of ATP synthase by a-KG causes conserved decrease in TOR pathway activity.
Figure 5, Panels a-h, shows that supplementation with a-KG extends C. *elegans* adult lifespan but does not change the growth rate of bacteria, or food intake, pharyngeal pumping rate or brood size of the worms. In Panel h, the first bars in each set is the vehicle.
Figure 6, Panels a-h, shows that a-KG binds to the b subunit of ATP synthase and inhibits the activity of Complex V but not the other ETC complexes.
Figure 7, Panels a-c, shows that treatment with oligomycin extends C. *elegans* lifespan and enhances autophagy in a manner dependent on *let-363.*
Figure 8, Panels a-b, shows analyses of oxidative stress in worms treated with a-KG or *atp-2* RNAi.
Figure 9, Panels a-c, shows lifespans of a-KG in the absence of *aak-2, daf-16, hif-1, vhl-1* or *egl-9.*
Figure 10, Panels a-f, shows a-KG decreases TOR pathway activity but does not directly interact with TOR.
Figure 11, Panels a-b, shows autophagy is enhanced in C. *elegans* treated with *ogdh-1* RNAi.
Figure 12 is a table showing enriched proteins in the α-KG DARTS sample. Only showing those proteins with at least 15 spectra in α-KG sample and enriched at least 1.5 fold.
Figure 13 is a table summarizing lifespan data from α-KG experiments.
Figure 14, Panels A-C, shows that 2-HG extends the lifespan of adult *C*. *elegans.*
Figure 15, Panels A-D, shows that 2-HG binds and inhibits ATP synthase. In Panel B, the top data points are vehicle, the middle data points are octyl (R)-2-HG, and the bottom data points are octyl (*S*)-2-HG.
Figure 16, Panels A-E, shows inhibition of ATP synthase in IDH1(R132H) cells.
Figure 17A shows U87/IDH1(R132H) cells have increased sensitivity to glucose starvation (***P < 0.001). The top line is IDH1(WT).
Figure 17B shows octyl α-KG treated U87 cells exhibit decreased viability upon glucose starvation (*****P* < 0.0001). On the right, the lines from top to bottom are 0 µM, 400 µM, and 800 µM.
Figure 17C shows octyl (*R*)-2-HG treated U87 cells exhibit decreased viability upon glucose starvation (****P* < 0.001). On the right, the lines from top to bottom are 0 µM, 400 µM, and 800 µM.
Figure 17D shows octyl (*S*)-2-HG treated U87 cells exhibit decreased viability upon glucose starvation (**P* < 0.05). On the right, the lines from top to bottom are 0 µM, 400 µM, and 800 µM.
Figure 17E shows ATP5B knockdown inhibits U87 cell growth (***P = 0.0004). The top line is control.
Figure 17F shows HCT 116 IDH1(R132H/+) cells exhibit increased vulnerability to glucose-free medium supplemented with (*R*)-3-hydroxybutyrate *(***P* < 0.001). By unpaired t-test, two-tailed, two-sample unequal variance. Mean ± s.d. is plotted in all cases. The top line is IDH1(+/+).
Figure 17G shows U87 cells with ATP5B knockdown exhibit decreased mTOR Complex 1 activity in glucose-free, galactose-containing medium.
Figure 17H shows U87 cells treated with membrane-permeable esterase-hydrolysable analogs of α-KG or 2-HG exhibit decreased mTOR Complex 1 activity in glucose-free, galactose-containing medium.
Figure 17I shows U87 cells stably expressing IDH1(R132H) exhibit decreased mTOR Complex 1 activity in glucose-free, galactose-containing medium.
Figure 18, Panels A-B, shows that 2-HG does not affect the electron flow through the electron transport chain and does not affect ADP import. In Panel A, the top data points are vehicle, the middle data points are octyl (R)-2-HG, and the bottom data points are octyl (S)-2-HG.
Figure 19, Panels A-C, shows that 2-HG inhibits cellular respiration.
Figure 20, Panels A-D, shows cellular energetics and metabolic profiles of 2-HG accumulated cells. In Panels C and D, the first bars in each set is the vehicle.
Figure 21, Panels A-B, shows that HCT 116 IDHI(R132H/+) cells exihibit metabolic vulnerability and growth inhibition. In Panel A,** is IDHI(R132H/+); and Panel B, ** is IDHI(R132H/+).
Figure 22, Panels A-E, shows cell growth inhibition upon ATP5B knockdown, treatment with octyl a-KG or octyl 2-HG, or IDHI(R132H) mutation. In Panel A,* is ATP5b siRNA; Panel B, *** is 400 µM and **** is 800 µM; Panel C, *** is 400 µM and** is 800 µM; Panel D, *** is 400 µMand** is 800 µM; and Panel E, ** is IDHI(R132H).
Figure 23 are graphs showing that octyl a-KG (200 µM) completely abolished (isoproterenol) ISO-induced hypertrophy (left panel), as well as suppressed ISO- and (phenylephrine) PE-induced overexpression (top right panel) of ANF (atrial natriuretic factor) (middle panel), and BNP (brain natriuretic peptide) (right panel).
Figure 24 is an OCR graph showing that mitochondria isolated from the hearts of a-KG-fed mice (SI, S2) exhibited lower state 3 respiration compared to that from control mice (Cl, C2). In State 3u, the data points from top to bottom are C2, Cl, S2, and S3.
Figure 25 are graphs showing the cardio-protective effect of a-KG *in vivo.*

Color versions of several of these drawings may be found in Chin, et al., (2014) Nature 510:397-401 and the Extended Data Figures related thereto.

### DETAILED DESCRIPTION OF THE INVENTION

In some embodiments, the present invention is directed to compositions for use in treating or inhibiting aging and age-related diseases in a subject, said compositions comprise at least one glutarate compound, at least one glutamate compound, or both. In some embodiments, the present invention is directed to compositions for increasing the lifespan of a subject, said compositions comprise at least one glutarate compound, at least one glutamate compound, or both. In some embodiments, the subject is an animal, which may or may not be an animal model of aging or an age-related disease. In some embodiments, the subject is a nematode, a rodent, or a non-human primate. In some embodiments, the subject is a human.

As used herein, "age-related diseases" refers to diseases and disorders often associated with aging and includes cancers (e.g., gliomas, leukemia, lymphoma, breast cancer, prostate cancer, lung cancer, and the like), neurodegenerative diseases (e.g., Parkinson's disease, Alzheimer's disease, Huntington's disease, dementia, and the like), sarcopenia, osteopenia, osteoporosis, arthritis, atherosclerosis, cardiovascular disease, hypertension, cataracts, presbyopia, glaucoma, type 2 diabetes, metabolic syndrome, alopecia, chronic inflammation, immunosenescence, age-related visual decline, age-related hair loss, thinning, and/or graying, and the like. As used herein, an "age-related heart condition" refers to cardiac hypertrophy, cardiomyopathy, heart failure, cardiac hypertrophy, cardiomyopathy, heart failure, and cardiovascular disease.

As used herein, methods and compositions that treat or inhibit "aging" in subjects are those that treat or inhibit symptoms related to aging. Symptoms related to aging include cancers, cholesterol build-up, stiffening of arterial wall, increased blood pressure, immunosenescence, muscle loss, bone loss, arthritis, osteoporosis, memory loss, hearing loss, visual decline, increased wrinkles, hair loss/thinning/graying, decreased stress resistance, dementia, loss of hearing, loss of vision, loss of mobility, loss of muscle strength and stamina, frailty, fatigue, increased susceptibility to infection, dry and/or wrinkled skin, and altered sleep patterns and circadian cycles.

As used herein, a "glutarate compound" refers to α-KG compounds, 2-HG compounds, and compounds having the following structural formula I: 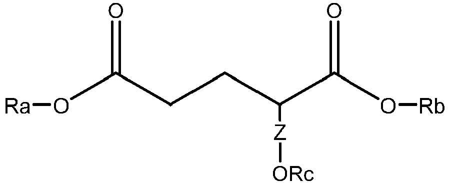 wherein
Ra and Rb are each independently a negative charge, H, a straight or branched C1-C10 alkyl, or a straight or branched C1-C10 alkenyl, and
Rc is optionally present, and if present, Rc is H, a straight or branched C1-C10 alkyl, or a straight or branched C1-C10 alkenyl, and if absent, Z is a double bond,
and pharmaceutically acceptable solvates, salts, prodrugs, and metabolites thereof.

As used herein, a "glutamate compound" refers to compounds having the following structural formula II: 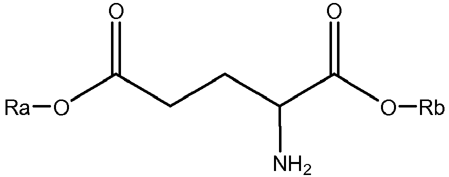 wherein
Ra and Rb are each independently a negative charge, H, a straight or branched C1-C10 alkyl, or a straight or branched C1-C10 alkenyl, and
and pharmaceutically acceptable solvates, salts, prodrugs, and metabolites thereof. Since α-KG can be produced anaplerotically from glutamate by oxidative deamination using glutamate dehydrogenase, and as a product of pyridoxal phosphate-dependent transamination reactions where glutamate is a common amino donor, glutamate is a prodrug of α-KG.

As used herein, a "C1-C10 alkyl" refers to an alkyl having 1-10 carbon atoms, and a "C1-C10 alkenyl" refers to an alkenyl having 1-10 carbon atoms.

As used herein, an "α-KG compound" refers to α-ketoglutarate (α-ketoglutarate), derivatives of α-ketoglutarate (e.g., the derivatives set forth in MacKenzie, et al. (2007) Mol Cell Biol 27(9):3282-3289)), analogues of α-ketoglutarate (e.g., phosphonate analogues (e.g., those recited in Bunik, et al. (2005) Biochemistry 44(31):10552-61), esters of α-ketoglutarate (e.g., dimethyl α-ketoglutarate and octyl α-ketoglutarate), and various species specific analogues, e.g., human α-ketoglutarate, porcine α- ketoglutarate, murine α-ketoglutarate , bovine α-ketoglutarate, and the like. As used herein, the abbreviation "KG" may be used to refer to the term "ketoglutarate", e.g., α-ketoglutarate is abbreviated as α-KG.

As used herein, a "2-HG compound" refers to 2-hydroxyglutaric acid, 2-hydroxypentanedioate, and compounds having 2-hydroxypentanedioate as part of its backbone structure and includes 1-alkyl-(*S*)-2-hydroxypentanedioate, 1-alkyl-(*R*)-2-hydroxypentanedioate, 1-alkenyl-(*S*)-2-hydroxypentanedioate, 1-alkenyl-(*R*)-2-hydroxypentanedioate, 5-alkyl-(*S*)-2-hydroxypentanedioate, 5-alkyl-(*R*)-2-hydroxypentanedioate, 5-alkenyl-(*S*)-2-hydroxypentanedioate, and 5-alkenyl-(*R*)-2-hydroxypentanedioate, wherein alkyl is a straight or branched C1-C10 alkyl and alkenyl is a straight or branched C1-C10 alkenyl. In some embodiments, the 2-HG compound is 1-octyl-(*S*)-2-hydroxypentanedioate, 1-octyl-(*R*)-2-hydroxypentanedioate, 5 -octyl-(*S*)- 2-hydroxypentanedioate, or 5-octyl-(*R*)-2-hydroxypentanedioate. As used herein, the abbreviation "HG" may be used to refer to the term "hydroxypentanedioate", e.g., 2-hydroxypentanedioate is abbreviated as 2-HG.

A "pharmaceutically acceptable solvate" refers to a solvate form of a specified compound that retains the biological effectiveness of such compound. Examples of solvates include compounds of the invention in combination with water, isopropanol, ethanol, methanol, dimethyl sulfoxide, ethyl acetate, acetic acid, ethanolamine, or acetone. Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of compounds of formulas I and II are within the scope of the invention. It will also be appreciated by those skilled in organic chemistry that many organic compounds can exist in more than one crystalline form. For example, crystalline form may vary from solvate to solvate. Thus, all crystalline forms of the compounds of formulas I and II or the pharmaceutically acceptable solvates thereof are within the scope of the present invention.

The term "pharmaceutically acceptable salts" refers to salt forms that are pharmacologically acceptable and substantially non-toxic to the subject being treated with the compound of the invention. Pharmaceutically acceptable salts include conventional acid-addition salts or base-addition salts formed from suitable non-toxic organic or inorganic acids or inorganic bases. Exemplary acid-addition salts include those derived from inorganic acids such as hydrochloric acid, hydrobromic acid, hydroiodic acid, sulfuric acid, sulfamic acid, phosphoric acid, and nitric acid, and those derived from organic acids such as p-toluenesulfonic acid, methanesulfonic acid, ethanedisulfonic acid, isethionic acid, oxalic acid, p-bromophenylsulfonic acid, carbonic acid, succinic acid, citric acid, benzoic acid, 2-acetoxybenzoic acid, acetic acid, phenylacetic acid, propionic acid, glycolic acid, stearic acid, lactic acid, malic acid, tartaric acid, ascorbic acid, maleic acid, hydroxymaleic acid, glutamic acid, salicylic acid, sulfanilic acid, and fumaric acid. Exemplary base-addition salts include those derived from ammonium hydroxides (e.g., a quaternary ammonium hydroxide such as tetramethylammonium hydroxide), those derived from inorganic bases such as alkali or alkaline earth-metal (e.g., sodium, potassium, lithium, calcium, or magnesium) hydroxides, and those derived from non-toxic organic bases such as basic amino acids.

"A pharmaceutically acceptable prodrug" is a compound that may be converted under physiological conditions or by solvolysis to the specified compound or to a pharmaceutically acceptable salt of such compound. "A pharmaceutically active metabolite" refers to a pharmacologically active product produced through metabolism in the body of a specified compound or salt thereof. Prodrugs and active metabolites of a compound may be identified using routine techniques known in the art. See, e.g., Bertolini, G. et al., (1997) J. Med. Chem. 40:2011-2016; Shan, D. et al., J. Pharm. Sci., 86(7):765-767; Bagshawe K., (1995) Drug Dev. Res. 34:220-230; Bodor, N., (1984) Advances in Drug Res. 13:224-331; Bundgaard, H., Design of Prodrugs (Elsevier Press, 1985) and Larsen, I. K., Design and Application of Prodrugs, Drug Design and Development (Krogsgaard-Larsen et al., eds., Harwood Academic Publishers, 1991).

In some embodiments, the amount of the glutarate compound administered to the subject is a therapeutically effective amount or an effective amount. As used herein, an "effective amount" is a dose that results in an observable difference as compared to a placebo. A "therapeutically effective amount", refers to an amount of one or more compounds of the present invention that, when administered to a subject, (i) treats or inhibits the particular disease, condition, or disorder, (ii) attenuates, ameliorates, or eliminates one or more symptoms of the particular disease, condition, or disorder, and/or (iii) inhibits or delays the onset of one or more symptoms of the particular disease, condition, or disorder, as compared to a control. A therapeutically effective amount of one or more compounds of the present invention will vary depending upon factors such as the given compound(s), the pharmaceutical formulation, route of administration, the type of disease or disorder, the degree of the disease or disorder, and the identity of the subject being treated, but can nevertheless be readily determined by one skilled in the art. For example, a "therapeutically effective amount" of a glutarate compound, a glutamate compound, or both is one that delays or inhibits the onset of age-related symptoms and/or extends the lifespan of a given subject as compared to one or more control subjects.

In some embodiments, a therapeutically effective amount of the one or more glutarate compounds and/or the one or more glutamate compounds is administered as a daily dose of about 0.25-2, about 0.5-2, about 1-2, or about 2 grams, per kilogram weight of the subject per day. The skilled artisan will appreciate that certain factors may influence the dosage required to effectively treat a subject, including but not limited to the severity of the disease or disorder, previous treatments, the general health and/or age of the subject, and other diseases present.

As shown herein, doses that increased α-KG levels in subjects by about 50% resulted in the largest increases in lifespan (up to about 70%). Therefore, in some embodiments, the amount of the one or more glutarate compounds and/or the one or more glutamate compounds administered to a subject is one that results in about a 50% increase in α-KG levels in the subject.

The therapeutically effective amount may be administered as a single dose or as multiple doses over a period of time. For example, a subject may be treated with one or more glutarate compounds and/or one or more glutamate compounds at least once. However, the subject may be treated with the one or more glutarate compounds and/or the one or more glutamate compounds from about one time per week to about once daily for a given treatment period. The length of the treatment period will depend on a variety of factors such as the severity of the disease or disorder, the concentration and activity of the one or more compounds of the present invention, or a combination thereof. It will also be appreciated that the effective dosage of the one or more compounds used for treatment may increase or decrease over the course of a particular treatment.

The one or more glutarate compounds and/or the one or more glutamate compounds to be administered to a subject may be provided as a pharmaceutical formulation. Pharmaceutical formulations may be prepared in a unit-dosage form appropriate for the desired mode of administration. The pharmaceutical formulations of the present invention may be administered by any suitable route including oral, rectal, nasal, topical (including buccal and sublingual), vaginal, and parenteral (including subcutaneous, intramuscular, intravenous, and intradermal). It will be appreciated that the route of administration may vary with the condition and age of the recipient, the nature of the condition to be treated, and the given compound(s) of the present invention. In some embodiments, the route of administration is oral. In some embodiments, the one or more glutarate compounds and/or the one or more glutamate compounds are provided in the form of a foodstuff.

It will be appreciated that the actual dosages of the glutarate compounds and/or the glutamate compounds used in the pharmaceutical formulations will vary according to the particular compound(s) being used, the particular composition formulated, the mode of administration, and the particular site, subject, and disease being treated. Optimal dosages for a given set of conditions may be ascertained by those skilled in the art using dosage determination tests in view of the experimental data for a given compound. Administration of prodrugs may be dosed at weight levels that are chemically equivalent to the weight levels of the fully active forms.

Pharmaceutical formulations of this invention comprise a therapeutically effective amount of one or more compounds of the present invention, and an inert, pharmaceutically acceptable carrier or diluent. As used herein the language "pharmaceutically acceptable carrier" is intended to include any and all solvents, dispersion media, coatings, antibacterial, and antifungal agents, isotonic and absorption delaying agents, and the like, compatible with pharmaceutical administration. The pharmaceutical carrier employed may be either a solid or liquid. Exemplary of solid carriers are lactose, sucrose, talc, gelatin, agar, pectin, acacia, magnesium stearate, stearic acid, and the like. Exemplary of liquid carriers are syrup, peanut oil, olive oil, water, and the like. Similarly, the carrier or diluent may include time-delay or time-release material known in the art, such as glyceryl monostearate or glyceryl distearate alone or with a wax, ethylcellulose, hydroxypropylmethylcellulose, methylmethacrylate, and the like. The use of such media and agents for pharmaceutically active substances is known in the art.

Toxicity and therapeutic efficacy of glutarate compounds and glutamate compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds exhibiting large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma may be measured, for example, by high performance liquid chromatography.

### α-KG extends the lifespan of adult C. elegans

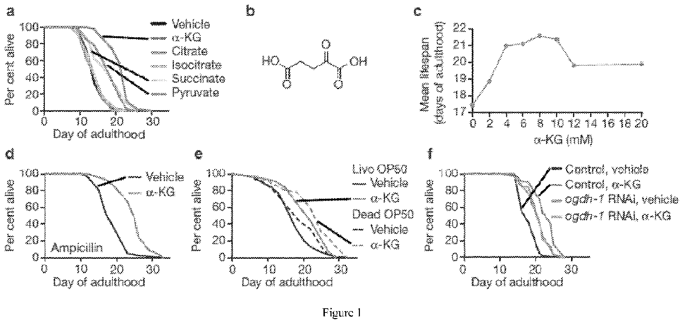

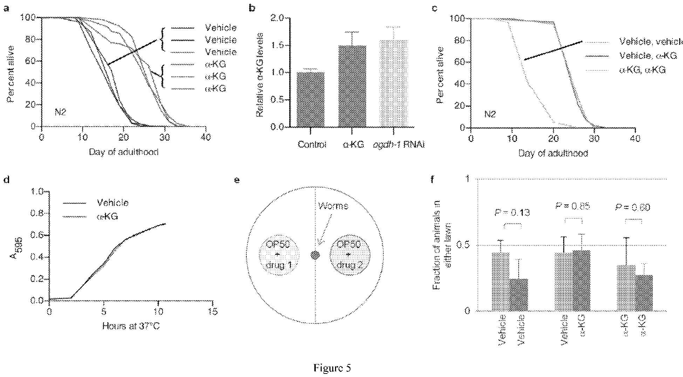

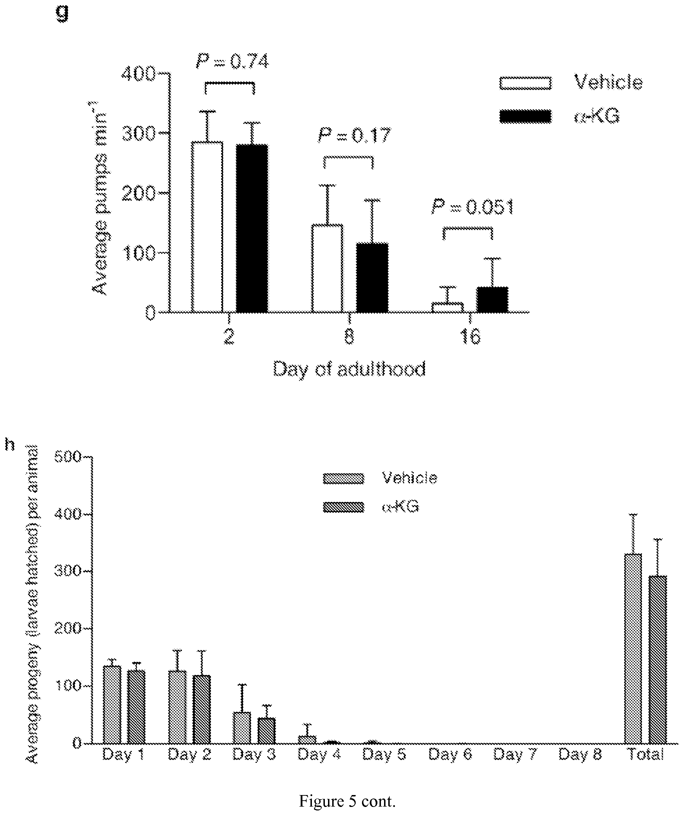

To gain insight into the regulation of aging by endogenous small molecules, normal metabolites and aberrant disease-associated metabolites were screened for their effects on the adult lifespan using the *C*. *elegans* model. It was discovered that the TCA cycle intermediate α-KG (but not isocitrate or citrate) delays aging and extends the lifespan of *C*. *elegans* by about 50% (Figure 1, Panel a, Figure 5, Panel a). In the cell, α-KG (or 2-oxoglutarate, Figure 1, Panel b) is produced from isocitrate by oxidative decarboxylation catalyzed by isocitrate dehydrogenase (IDH). α-KG extended wild-type N2 lifespan in a concentration-dependent manner, with 8 mM α-KG producing the maximal lifespan extension (Figure 1, Panel c); 8 mM was the concentration used in all subsequent *C*. *elegans* experiments. There is a about a 50% increase in α-KG concentration in worms on 8 mM α-KG plates compared to those on vehicle plates (Figure 5, Panel b), or about 160 µM versus about 110 µM assuming homogenous distribution. α-KG not only extends lifespan, but also delays age-related phenotypes, such as the decline in rapid, coordinated body movement (Supplementary Videos 1 and 2, available on the internet at WorldWideWeb.natureDOTCOM/nature/journal/v510/n7505/fig_tab/nature13264_SV1. HyperTextMarkupLanguage and WorldWideWeb.natureDOTCOM/nature/journal/v510/n7505/fig_tab/nature13264_SV2. HyperTextMarkupLanguage, wherein "WorldWideWeb" is "www", "DOTCOM" is ".com", and "HyperTextMarkupLanguage" is "html"). α-KG supplementation in the adult stage is sufficient for longevity (Figure 5, Panel c).

The dilution or killing of the bacterial food has been shown to extend worm lifespan, but the lifespan increase by α-KG is not due to altered bacterial proliferation, viability, or metabolism (Figure 1, Panels d-e, Figure 5, Panel d). Animals also did not view α-KG-treated food as less favorable (Figure 5, Panels e-f), and there was no significant change in food intake, pharyngeal pumping, foraging behavior, body size, or brood size in the presence of α-KG (Figure 5, Panels e-h, data not shown).

In the cell, α-KG is decarboxylated to succinyl-CoA and CO₂ by α-KG dehydrogenase (encoded by *ogdh-1*), a key control point in the TCA cycle. Increasing α-KG levels by *ogdh-1* RNAi (Figure 5, Panel b) also extends worm lifespan (Figure 1, Panel f), consistent with a direct effect of α-KG on longevity independent of the bacterial food.

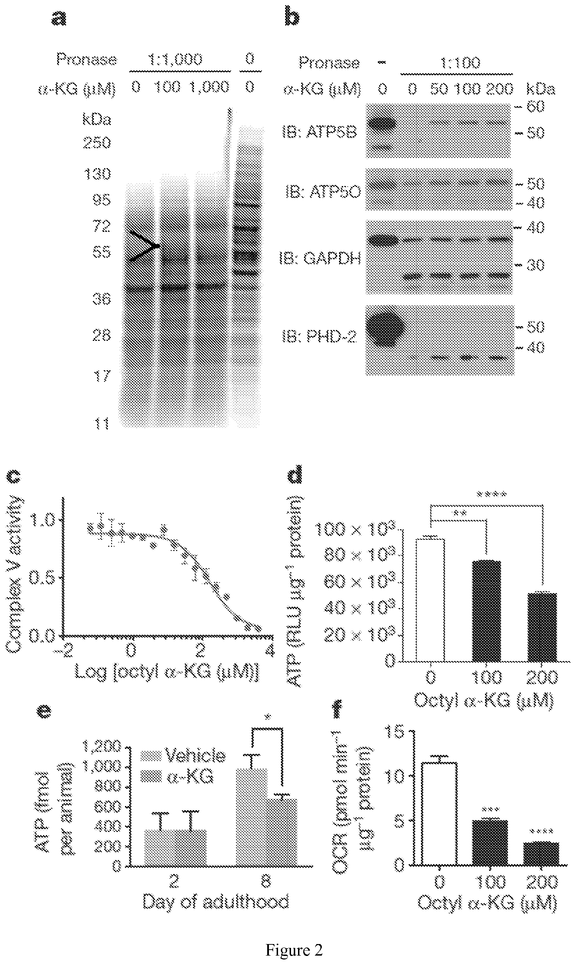

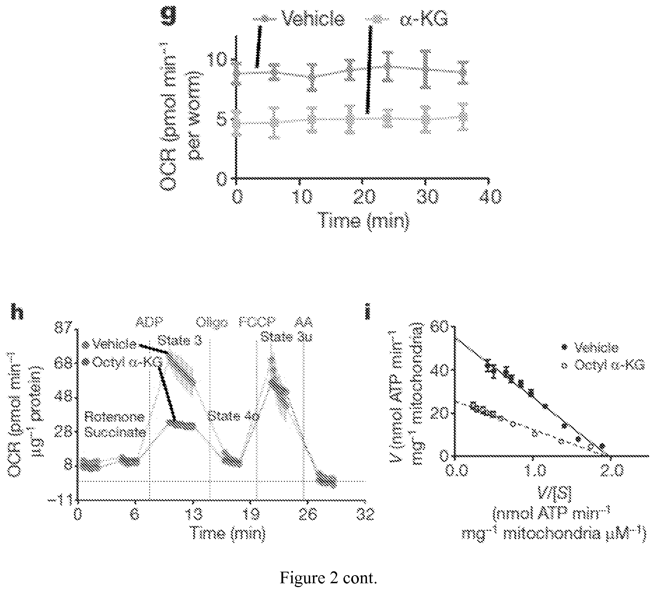

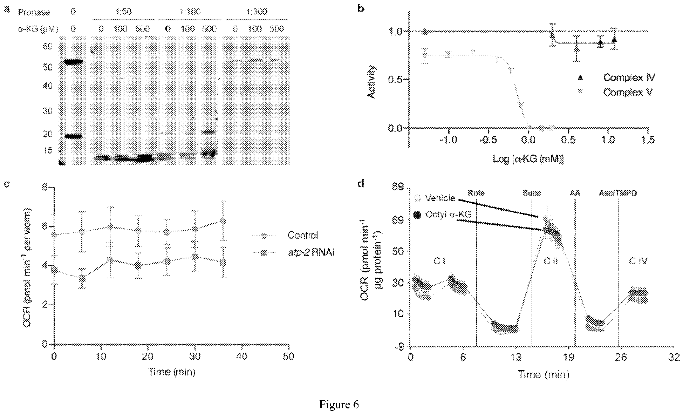

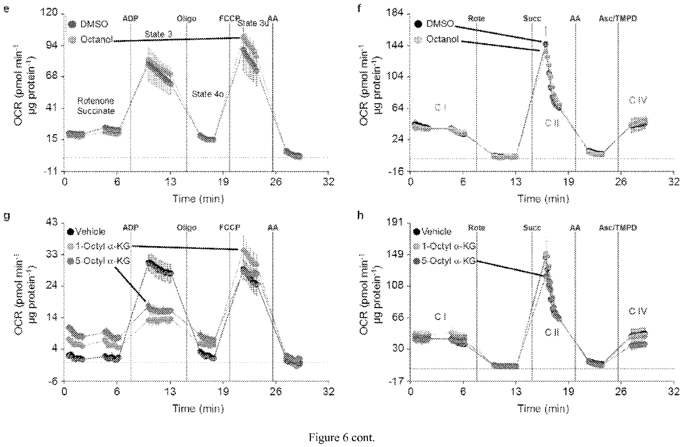

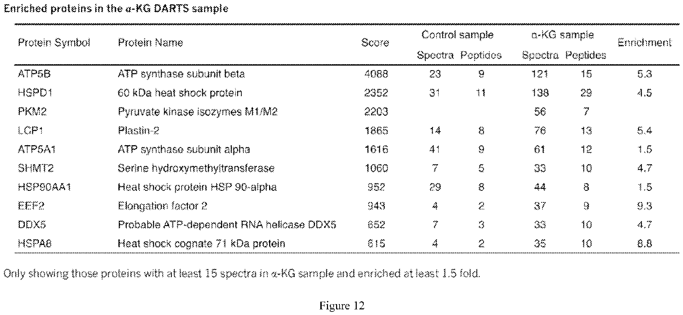

To investigate the molecular mechanism(s) of longevity by α-KG, the unbiased biochemical approach DARTS was used. A human cell line (Jurkat), which is easy to culture, was used as the protein source for DARTS (Figure 2, Panel a). Mass spectrometry identified ATP5B, the beta subunit of the catalytic core of the ATP synthase, among the most abundant and enriched proteins present in the α-KG treated sample (Figure 12); the homologous alpha subunit ATP5A was also enriched albeit to a lesser extent. The interaction between α-KG and ATP5B was verified using additional cell lines (Figure 2, Panel b, data not shown), and corroborated for the *C*. *elegans* ortholog ATP-2 (Figure 6, Panel a).

α-KG inhibits the activity of Complex V, but not Complex IV, from bovine heart mitochondria (Figure 2, Panel c, Figure 6, Panel b, data not shown). This inhibition is also readily detected in live mammalian cells (Figure 2, Panel d, data not shown) and in live nematodes (Figure 2, Panel e), as evidenced by reduced ATP levels. Concomitantly, oxygen consumption rates are lowered (Figure 2, Panels f-g), similar to the scenario with *atp-2* knockdown (Figure 6, Panel c). Specific inhibition of Complex V, but not the other ETC complexes, by α-KG is further confirmed by respiratory control analysis (Figure 2, Panel h, Figure 6, Panels d-h).

To understand the mechanism of inhibition by α-KG, the enzyme inhibition kinetics of ATP synthase was studied. α-KG (released from octyl α-KG) decreases both the effective *V*ₘₐₓ and *K*ₘ of ATP synthase, indicative of uncompetitive inhibition (Figure 2, Panel i).

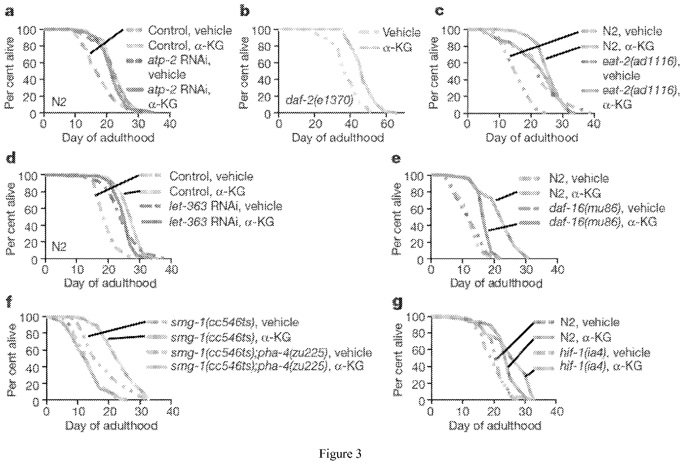

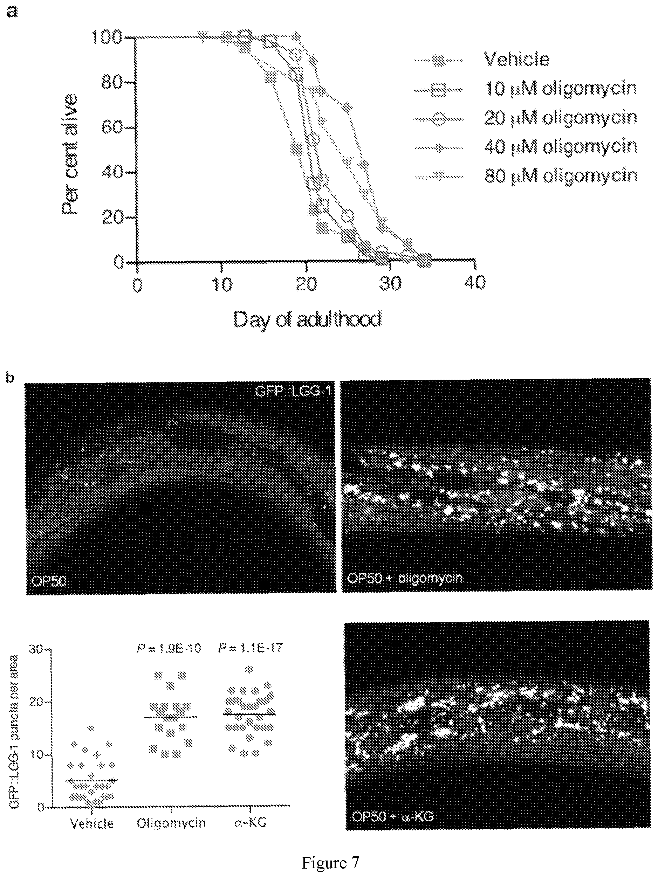

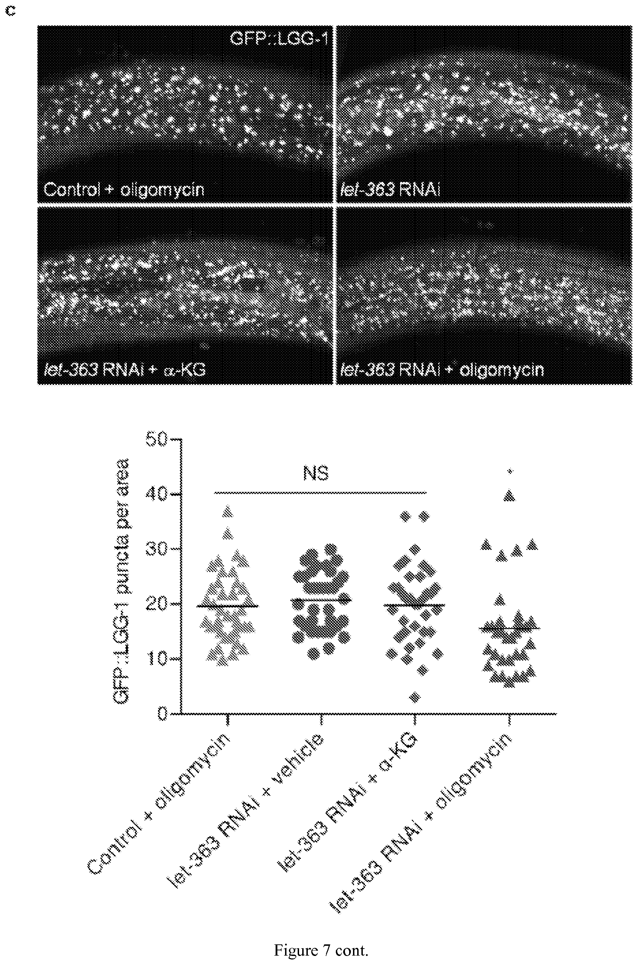

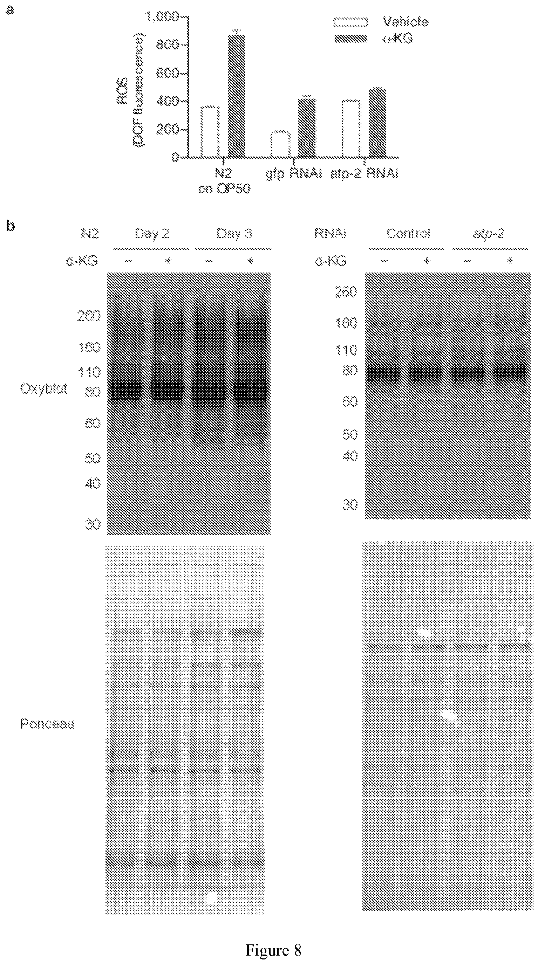

To determine the significance of ATP-2 to the longevity by α-KG, the lifespan of *atp-2(RNAi)* adults given α-KG was measured. *atp-2(RNAi)* animals live longer than controls (Figure 3, Panel a). However, their lifespan is not further extended by α-KG (Figure 3, Panel a), indicating that ATP-2 is involved in the longevity benefit of α-KG. In contrast, the lifespan of the even longer-lived insulin/IGF-1 receptor *daf-2(e1370)* mutant worms is further increased by α-KG (Figure 3, Panel b). Remarkably, oligomycin, an inhibitor of ATP synthase, also extends the lifespan of adult worms (Figure 7, Panel a). Together, the direct binding of ATP-2 by α-KG, the related enzymatic inhibition, reduction in ATP levels and oxygen consumption, lifespan analysis, and other similarities (see also Figure 8) to *atp-2* knockdown or oligomycin treatment demonstrate that α-KG likely extends lifespan by targeting ATP-2.

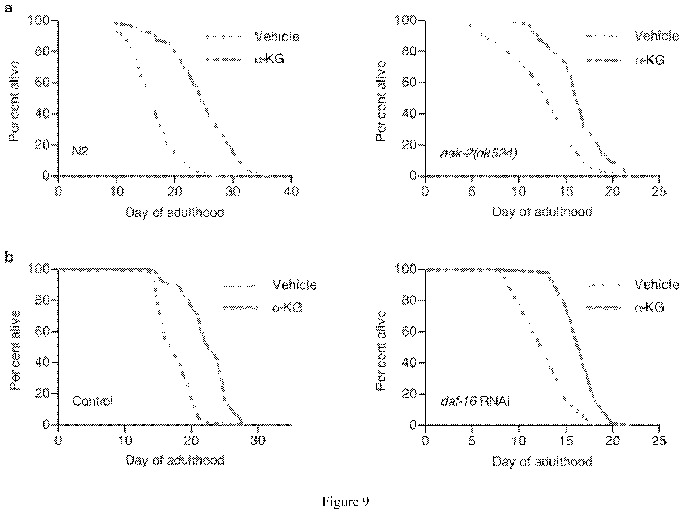

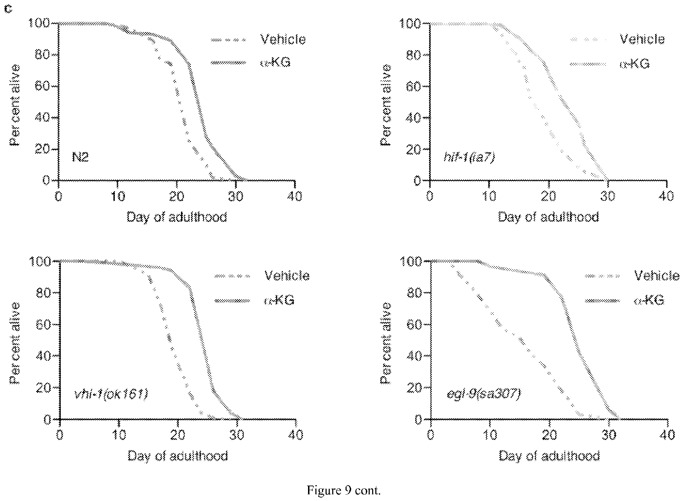

It was found that α-KG does not extend the lifespan of *eat-2(ad1116)* animals (Figure 3, Panel c), which is a model of DR with impaired pharyngeal pumping and therefore reduced food intake. The longevity of *eat-2* mutants involves TOR/*let-363*, an important mediator of the effects of DR on longevity. Likewise, α-KG does not increase the lifespan of *CeTOR(RNAi)* animals (Figure 3, Panel d). The AMP-activated protein kinase (AMPK) is another conserved major sensor of cellular energy status. Both AMPK/*aak-2* and the FoxO transcription factor DAF-16 mediate DR-induced longevity in *C*. *elegans* fed diluted bacteria, but neither is required for lifespan extension in the *eat-*2 model. It was found that, in *aak-2* (Figure 9, Panel a) and *daf-16* (Figure 3, Panel e) mutants, the longevity effect of α-KG is smaller than in N2 (*P* < 0.0001), suggesting that α-KG longevity partially involves AMPK and FoxO; nonetheless, lifespan is significantly increased by α-KG in *aak-2* (24.3%, *P* < 0.0001) and *daf-16* (29.5%, *P <* 0.0001) mutant or RNAi animals (Figure 3, Panel e, Figure 9, Panels a-b, data not shown), indicating an AMPK-FoxO independent effect by α-KG in longevity.

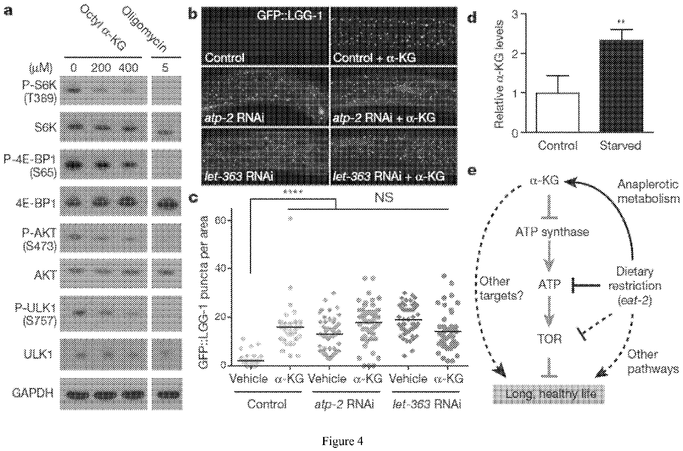

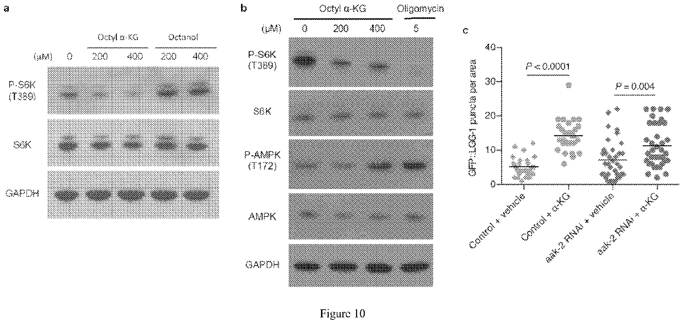

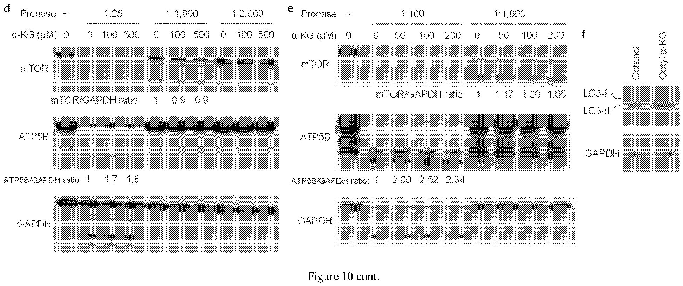

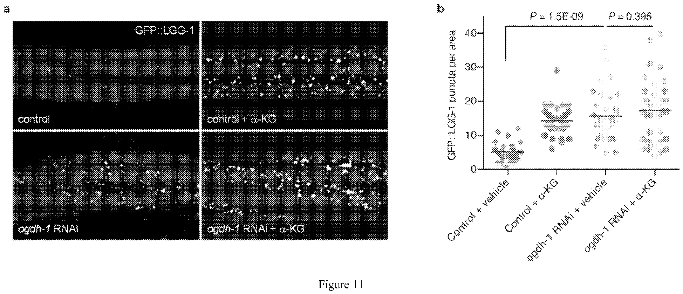

The inability of α-KG to further extend the lifespan of *CeTOR(RNAi)* animals indicates that α-KG treatment and TOR inactivation extend lifespan either through the same pathway (with α-KG acting on or upstream of TOR), or through independent mechanisms or parallel pathways that converge on a downstream effector. The first model predicts that the TOR pathway will be less active upon α-KG treatment, whereas if the latter model were true then TOR would be unaffected by α-KG treatment. In support of the first model, it was found that TOR pathway activity is decreased in human cells treated with octyl α-KG (Figure 4, Panel a, Figure 10, Panels a-b). However, α-KG does not interact with TOR directly (Figure 10, Panels d-e). Consistent with the involvement of TOR in α-KG longevity, the FoxA transcription factor PHA-4 is likewise involved in α-KG-induced longevity (Figure 3, Panel f). Moreover, autophagy, which is activated both by TOR inhibition and by DR, is markedly increased in worms treated with α-KG (or *ogdh-1* RNAi) and in *atp-2(RNAi)* animals (Figure 4, Panels b-c, Figure 10, Panel c, Figure 11), as indicated by the prevalence of GFP::LGG-1 puncta. Autophagy was also induced in mammalian cells treated with octyl α-KG (Figure 10, Panel f). Furthermore, α-KG does not result in significantly more autophagy in either *atp-2(RNAi)* or *CeTOR(RNAi)* worms (Figure 4, Panels b-c). The data provide further evidence that α-KG decreases TOR pathway activity through the inhibition of ATP synthase. Similarly, autophagy is induced by oligomycin, and oligomycin does not augment autophagy in *CeTOR(RNAi)* worms (Figure 7, Panels b-c).

α-KG is not only a metabolite, but also a co-substrate for a large family of dioxygenases. The hypoxia inducible factor (HIF-1) is modified by one of these enzymes, the prolyl 4-hydroxylase (PHD) EGL-9, and thereafter degraded by the von Hippel-Lindau (VHL) protein. It was found that α-KG extends the lifespan of animals with loss-of-function mutations in *hif-1, egl-9*, and *vhl-1* (Figure 3, Panel g, Figure 9, Panel c), and thereby suggests that this pathway does not play a major role in lifespan extension by α-KG. Nevertheless, other α-KG binding targets may also play a role in the extension of lifespan.

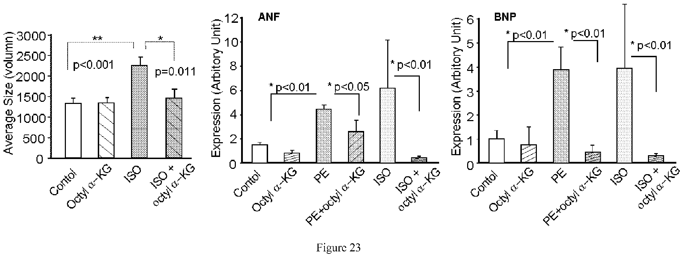

The protective effects of octyl α-KG against isoproterenol-induced hypertrophy in isolated neonatal rat cardiomyocytes was examined. Cardiomyocytes of neonatal rats were isolated by collagenase digestion and cultured overnight in DMEM containing 10% fetal calf serum (FCS), and then culture medium was changed to serum-free high glucose insulin-transferrin-sodium selenite (ITS). Hypertrophy of cardiomyocytes was induced by treating the cells with 1 mM isoproterenol (ISO) or phenylephrine (PE) for 48 hours. As shown in Figure 23, octyl α-KG (200 µM) completely abolished ISO-induced hypertrophy (left panel), as well as suppressed ISO- and PE-induced overexpression (right panel) of the hypertrophy associated markers, atrial natriuretic factor (ANF) and brain natriuretic peptide (BNP), indicating a cardio-protective and anti-hypertrophy effect by α-KG.

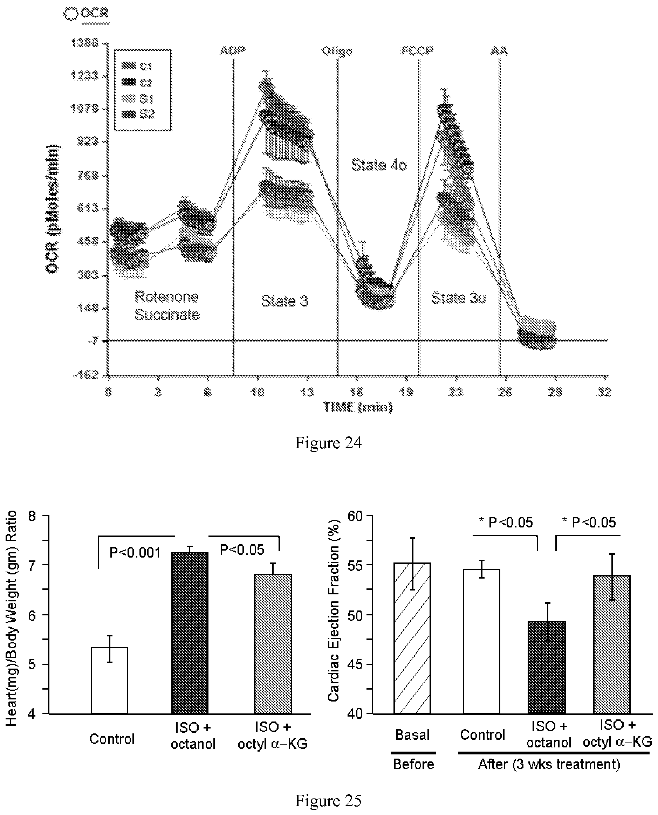

To test the bio- and oral availability of octyl α-KG in animals, animals were fed octyl α-KG and assessed whether the molecular and cellular effects of octyl α-KG could be recapitulated *in vivo,* particularly its inhibition of mitochondrial ATP synthase (Complex V) activity. Mice were fed a chow diet that was pre-mixed with either octyl α-KG (1.5 mg/g body weight) or octanol (control) for one week. The animals showed no abnormality either physiologically or behaviorally after 5 days feeding with octyl α-KG or octanol. The mice were sacrificed, hearts harvested, and mitochondria isolated. The oxygen consumption rate (OCR) was measured using a Seahorse XF-24 Analyzer (Seahorse Bioscience). As shown in Figure 24, the mitochondria isolated from α-KG-fed mice (S1, S2) exhibited lower state 3 respiration compared to that from control mice (C1, C2), mirroring the effects of octyl α-KG on isolated mitochondria directly. The decrease in the state 3 respiration in octyl α-KG fed mice indicates that octyl α-KG can be taken up, absorbed, and distributed in the body to release α-KG to act on its cellular target.

The cardio-protective effect of α-KG *in vivo* was examined. Hypertrophy and heart failure were induced by chronic infusion of isoproterenol for 3 weeks at a dose of 30 µg per gram bodyweight per day using osmotic mini-pumps (Alzet, model 1004). DBA2/J female mice at 8 weeks old were used for this study. The mice were fed octyl α-KG at 0.5 mg/g body weight daily in chow during the three weeks of the study. ISO-induced cardiac hypertrophy and cardiomyopathy were determined at the end of experiment by assessing heart size and heart to body weight ratio. As shown in the left panel of Figure 25, at the end of experiment, the heart (mg)/body (g) ratios are 5.32 ± 0.26 (n=4) for the control group, 7.26 ± 0.12 (n=3) for ISO-and octanol treated group, and 6.81 ± 0.24 (n=6) (Mean, STEDV) for the ISO and octyl α-KG treated group. ISO-treated mice displayed a marked increase in heart size and heart to body weight ratio, whereas ISO-treated mice when also treated with octyl α-KG exhibited significantly reduced heart size and heart to body weight ratio.

More importantly, cardiac output was assessed and the cardiac ejection fractions were determined by echocardiography. The cardiac EF for the basal levels before the treatment is 55.1% ± 2.6 (n=14). As shown in the right panel of Figure 25, at the end of the study (3 weeks), the cardiac EFs were 54.5%+0.9 for the no-ISO control group (n=2), 49.2% ± 1.9 for the ISO-treated group (with octanol) (n=3), and 53.8% ± 2.3 for ISO plus octyl α-KG treated (n=6). The EF for the control group (No-ISO, plus octanol) after three weeks is comparable to the basal before the treatment. The EF of ISO-treated is significantly reduced compared to the no-ISO group. Remarkably, the EF of the ISO plus octyl α-KG is restored to the levels of the no-ISO group. These results show that α-KG can significantly reduce ISO-induced cardiac hypertrophy and restore the cardiac output in an experimental heart failure animal model.

### 2-HG extends the lifespan of adult C. elegans

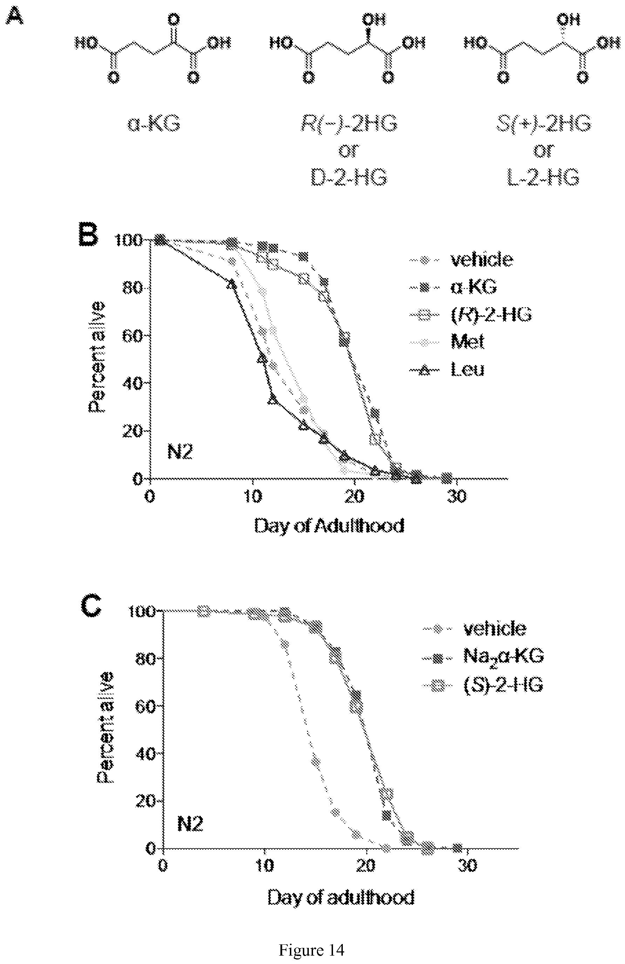

Although 2-HG exhibits structural similarity with α-KG (Figure 14, Panel A), 2-HG is associated with neurological disorders, cancer, and various age-related diseases. Therefore, various experiments with 2-HG were conducted to determine whether 2-HG causes decreased longevity. Surprisingly, both (R)-2-HG and (S)-2-HG increase the lifespan of *C*. *elegans* to a comparable extent as α-KG (Figure 14, Panel B-C).

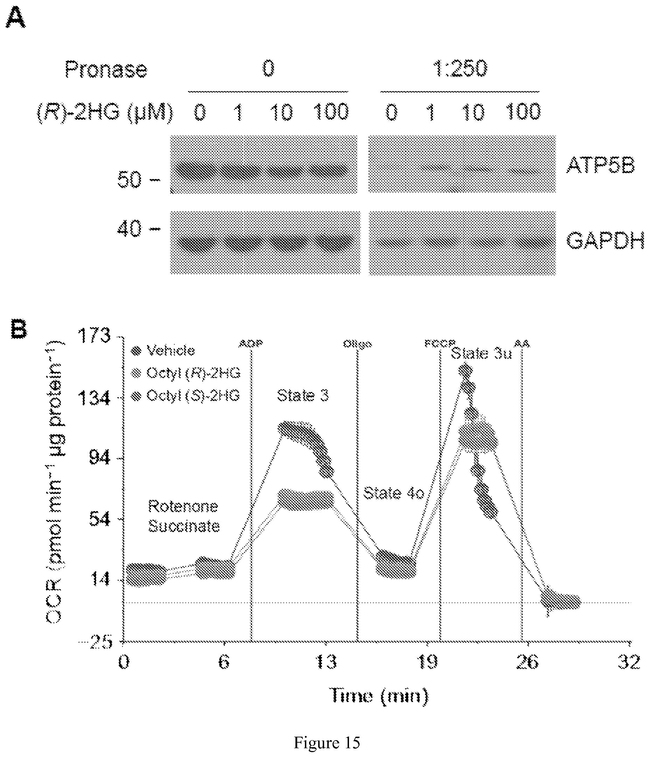

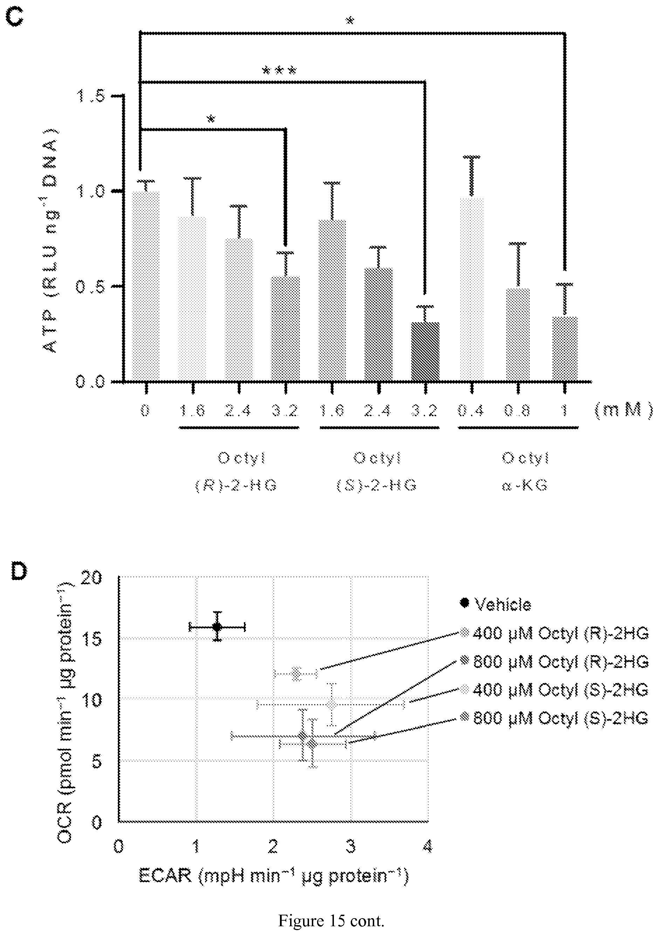

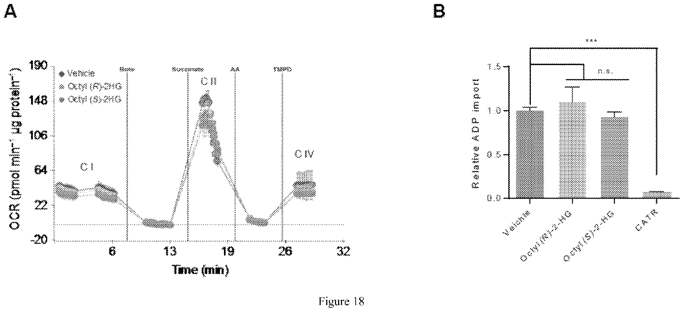

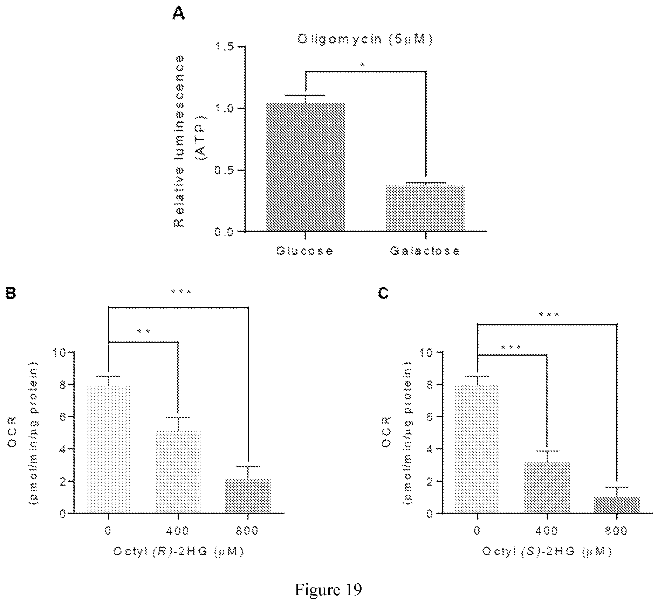

DARTS analysis shows that 2-HG targets ATP5B. Specifically, it was found that both (R)-2-HG and (S)-2-HG bind to ATP5B (Figure 15, Panel A, and data not shown). Like α-KG, 2-HG inhibits ATP synthase (Complex V) (Figure 15, Panel B). This inhibition is specific as 2-HG does not inhibit other electron transport chain (ETC) complexes (Figure 18, Panel A) or ADP import into the mitochondria (Figure 18, Panel B). The inhibition of ATP synthase by 2-HG is also readily detected in live cells; treatment of U87 glioblastoma cells (wild-type IDH1/2) with membrane-permeable octyl esters of α-KG or 2-HG results in decreased cellular ATP content under mitochondrial oxidative phosphorylation conditions (Figure 15, Panel C), as with the well-established ATP synthase inhibitor oligomycin (Figure 19, Panel A). Both total and ATP synthase-linked oxygen consumption rates are decreased in the treated cells (Figure 15, Panel D and Figure 19, Panel B-C).

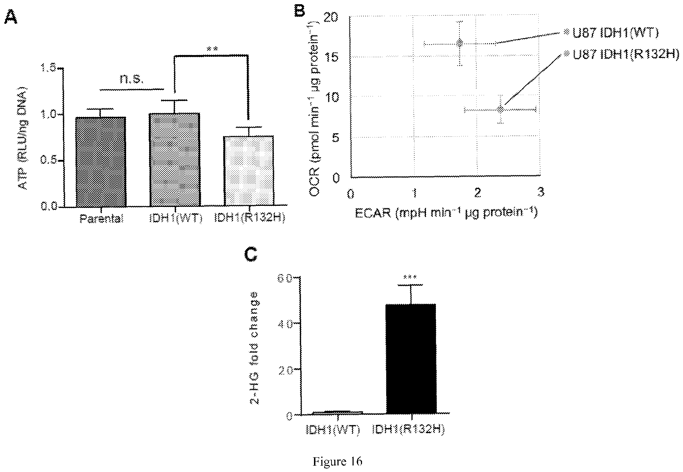

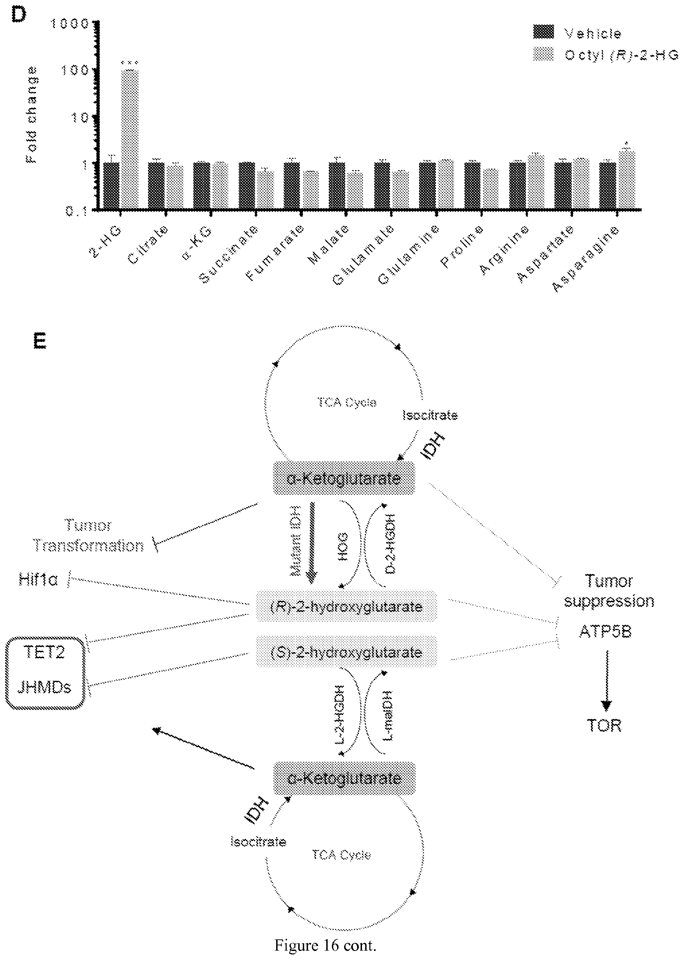

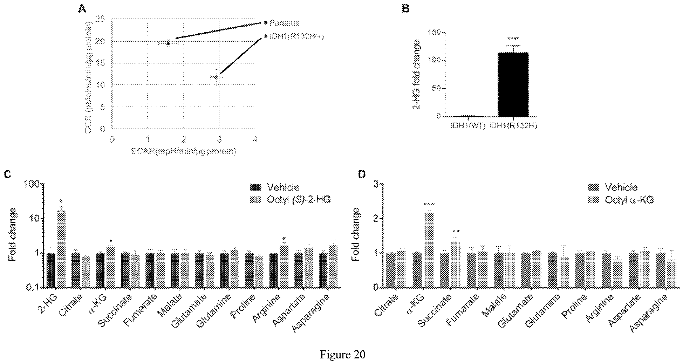

At normal cellular concentrations of about 200 µM, (R)-2-HG is unlikely to cause significant inhibition of ATP synthase. However, in glioma patients with the IDH1 or IDH2 mutations where (*R*)-2-HG accumulates to 10-100 times natural endogenous levels, inhibition of ATP synthase would be possible. To test this idea, U87 cells stably expressing IDH1(R132H), the most common IDH mutation in glioma, were used. Similar to octyl 2-HG treated cells described above, the U87/IDH1(R132H) cells exhibit decreased ATP content and oxygen consumption rates compared to isogenic IDH1(WT)-expressing U87 cells (Figure 16, Panels A-B). Similar results were obtained in HCT 116 IDH1(R132H/+) cells (Figure 20, Panel A). The intracellular (*R*)-2-HG levels are about 50-100 fold higher in the U87 and HCT 116 cells expressing IDH1 (R132H) than control cells (Figure 16, Panel C, and Figure 20, Panel B), and are comparable to the increase in (*R*)-2-HG levels found in cells with octyl (*R*)-2-HG treatment (Figure 16, Panel D) and IDH1-mutant tumor samples. These data are consistent with the inhibition of ATP synthase and mitochondrial respiration by (*R*)-2-HG in mutant IDH1 cancer cells.

The metabolite 2-HG is linked to the TCA cycle and related amino acid metabolic pathways (Figure 16, Panel E). To explore potential metabolic changes upon octyl 2-HG treatment, metabolite levels in octyl 2-HG treated cells were measured by LC-MS. It was found that 2-HG is accumulated to about 20-100 fold after octyl 2-HG treatment (Figure 16, Panel D, and Figure 20, Panel C). Compared to the accumulation of 2-HG, there is no dramatic change (< 2-fold) in TCA cycle metabolites or related amino acids (Figure 16, Panel D, and Figure 20, Panel C). Similarly, in octyl α-KG treated samples, the accumulation of α-KG is the most profound change in metabolic profile (Figure 20, Panel D). The static metabolic profile supports the notion that the bioenergetic shift (and signaling change, see below) observed in 2-HG (or α-KG) treated cells result from the direct inhibition of ATP synthase by 2-HG (or α-KG) rather than global metabolic effects.

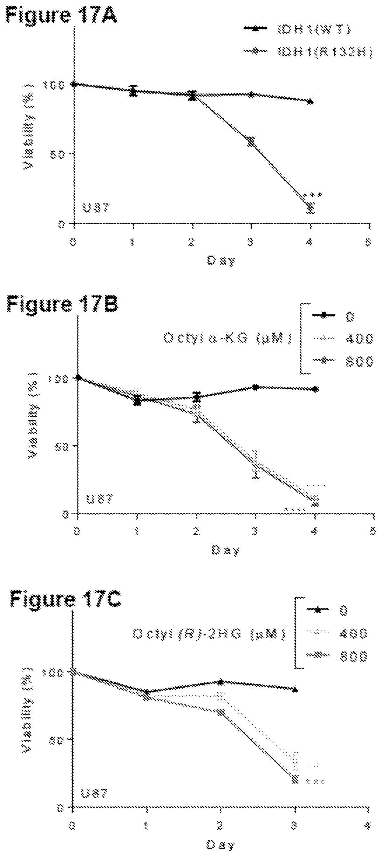

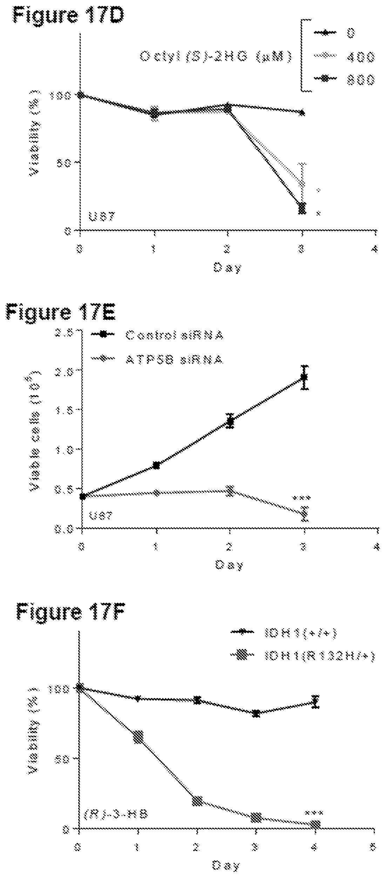

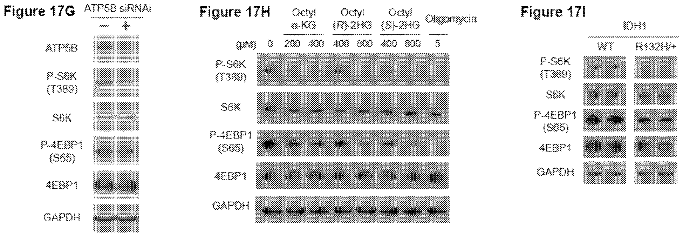

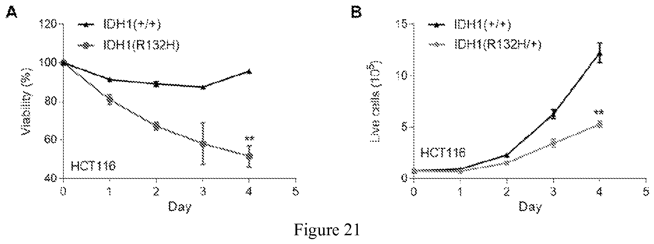

As the end component (Complex V) of the mitochondrial electron transport chain (ETC), ATP synthase is a major source of cellular energy and the sole site for oxidative phosphorylation. When glycolysis is inhibited, such as under conditions of glucose insufficiency, cells are forced to rely on mitochondrial respiration as a source of ATP. The inherent inhibition of ATP synthase and mitochondrial respiration in mutant IDH1 cancer cells thus suggests a possible Achilles' heel for these cancers. Supporting this idea, when cultured in glucose-free, galactose-containing medium, e.g., when respiration is the primary source of energy, IDH1(R132H) cells exhibit drastically decreased cell viability (Figure 17, Panel A and Figure 21, Panel A). These results indicate a special sensitivity of IDH1(R132H) mutant cells to the deprivation of glucose. The mutant cell line is not sensitive to FBS deprivation (data not shown), indicating its increased vulnerability to glucose starvation is specific. A similar metabolic vulnerability is evident in U87 cells treated with octyl α-KG or octyl 2-HG (Figure 17, Panels B-D) and in ATP5B knockdown cells (Figure 17, Panel E). These findings indicate that cancer cells with the IDH1(R132H) mutation, due to the inability to utilize mitochondrial respiration as a result of ATP synthase shut down, may also be particularly sensitive to nutrient conditions analogous to glucose limitation.

In complex organisms, glucose limitation can be achieved through ketosis, wherein cells use ketone bodies (instead of glucose) for energy. Ketosis is naturally induced upon prolonged starvation (or fasting), in a survival mode for the body to derive energy from its relatively long-lasting fat reservoir while sparing protein in muscle and other tissues from catabolism. Ketosis can also be implemented through a low-carbohydrate-high-fat "ketogenic diet", which has shown benefits against cancer. One reason for this may be that tumor cells largely depend on glucose for growth and survival. Since metabolism of ketone bodies depends entirely on mitochondrial oxidative phosphorylation, one prediction is that inhibiting ATP synthase (or other ETC components) in cancer cells would confer a survival disadvantage if ketone bodies were to be their only source of energy. Since U87 cells are unable to utilize ketone bodies for energy, in order to directly assess the effect of the IDH1 mutation HCT 116 cells expressing the mutant IDH1 were used. When cultured in ketogenic (glucose-free) medium containing the ketone body (R)-3-hydroxybutyrate, IDH1 mutant HCT 116 cells showed a profound decrease in viability compared to the parental cells (Figure 17, Panel F), confirming the purported metabolic weakness of IDH mutant cells. These results not only further support the finding that 2-HG accumulation in mutant IDH cancer cells results in ATP synthase inhibition, but also suggest novel metabolic therapeutic strategies in cancer treatment.

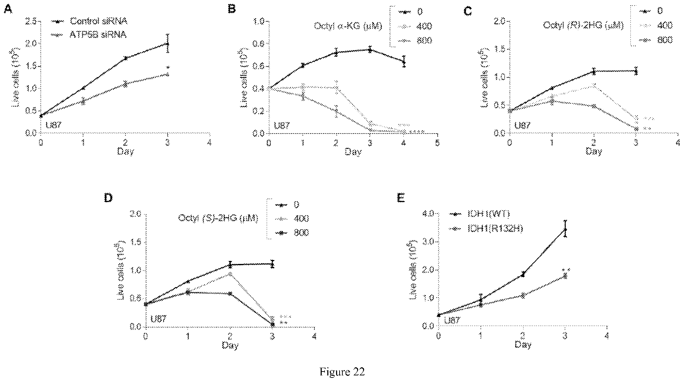

Similar to treatment with ATP synthase inhibitors, octyl α-KG or oligomycin, which decreases TOR signaling, it was found that phosphorylation of mTOR Complex 1 substrates, including S6K and 4E-BP1, is decreased in ATP5B knockdown cells (Figure 17, Panel G), in cells treated with octyl esters of 2-HG (Figure 17, Panel H), and in IDH1(R132H) expressing cells (Figure 17, Panel I). These results indicate that inhibition of ATP synthase leads to decreased mTOR activity in the cells, consistent with results obtained in worms and flies. As TOR is a major regulator of cell growth, decreased mTOR pathway activity in ATP synthase-inhibited cells predicts a possible cell growth arrest. Indeed, growth is completely arrested when ATP5B knockdown cells are cultured with galactose as a sole sugar source, e.g., when they are forced to rely on mitochondrial respiration for ATP production (Figure 17, Panel E). Even in glucose medium, decreased cell growth is readily detectable (Figure 22, Panel A). Treatment with octyl α-KG or octyl 2-HG similarly inhibits U87 cell growth (Figure 22, Panels B-D). Growth inhibition by 2-HG (and by α-KG) is also observed in other cancer cell lines tested, including A549 and Jurkat, in immortalized non-malignant HEK 293 cells, and in normal human diploid fibroblasts WI-38 (data not shown), suggesting that the mechanism for growth inhibition may be universal and that in excess 2-HG may serve as a growth inhibitory metabolite. A similar growth inhibition is evident in both U87 and HCT 116 cells expressing mutant IDH1(R132H) (Figure 22, Panel E and Figure 21, Panel B). These data together suggest a consistent inhibition status of ATP synthase and mTOR in mutant IDH1 cells.

In summary, similar to α-KG, both enantiomers of 2-HG bind and inhibit ATP synthase and extend the lifespan of *C*. *elegans.* Inhibition of ATP synthase by these related metabolites decreases mitochondrial respiration and mTOR signaling. Both 2-HG and α-KG exhibit broad growth-inhibitory effects and reduce cancer cell viability in glucose limiting conditions. The experiments herein suggest that although 2-HG is an oncometabolite interfering with various α-KG binding factors with importance in cancer, 2-HG also acts - through inhibition of ATP synthase and mTOR signaling downstream - to decrease tumor cell growth and viability.

The following examples are intended to illustrate but not to limit the invention.

### EXAMPLES

**Nematode strains and maintenance.** *Caenorhabditis elegans* strains were maintained using methods known in the art. The following strains were used:

| Strain | Genotype | Source |
|---|---|---|
| Bristol N2 | wild-type | *Caenorhabditis* Genetics Center (CGC), University of Minnesota |
| DA1116 | *eat-2(ad1116)*II | CGC |
| CB1370 | *daf-2(e1370)*III | CGC |
| CF1038 | *daf-16(mu86)*I | CGC |
| PD8120 | *smg-1(cc546ts)*I | CGC |
| SM190 | *smg-1(cc546ts)*I;*pha-4(zu225)*V | CGC |
| RB754 | *aak-2(ok524)X* | CGC |
| ZG31 | *hif1(ia4)V* | CGC |
| ZG596 | *hif1(ia7)V* | CGC |
| JT307 | *egl-9(sa307)V* | CGC |
| CB5602 | *vhl-1(ok161)X* | CGC |
| DA2123 | *adls2122[lgg-1::GFP* + *rol-6(su1006)]* | CGC |

### Cell Culture

U87 cells were cultured in glucose-free DMEM (Life technologies, 11966-025) supplemented with 10% fetal bovine serum (FBS) and 10 mM glucose, or in glucose-free DMEM supplemented with 10% FBS and 10 mM galactose when indicated. IDH1(R132H) mutant or IDH1(WT) expressing U87 cells were as reported (Li, et al. Neuro Oncol 15, 57-68). Normal human diploid fibroblasts WI-38 (ATCC, CCL-75) were cultured with EMEM (ATCC, 30-2003) supplemented with 10% FBS. HEK 293, A549, and HeLa cells were cultured with DMEM (Life technologies, 11966-065) supplemented with 10% FBS. Jurkat and HCT 116 cells were cultured in RPMI (Life technologies, 11875-093) supplemented with 10% FBS. All the cells were cultured at 37°C and 5% CO₂. Cells were transfected with indicated siRNA using Thermo Scientific DharmaFECT Transfection Reagent 1 by following the manufacturer's instructions. Knock down efficiency was confirmed by immunoblotting on the first and the last day of the growth inhibition assay.

### Compounds

1-octyl α-KG, octyl (*S*)-2-HG, and octyl (R)-2-HG were synthesized using methods known in the art. See Jung & Deng, J Org Chem 77, 11002-11005 (2012); Albert et al. Synthesis-Stuttgart, 635-637 (1987); and Cancer Cell 19, 17-30 (2011). Modifications to synthetic methods are as follows:

### Synthesis of octyl α-KG

Briefly, 1-octanol (0.95 ml, 6.0 mmol), DMAP (37 mg, 0.3 mmol), and DCC (0.743 g, 3.6 mmol) were added to a solution of 1-cyclobutene-1-carboxylic acid (0.295 g, 3.0 mmol) in dry CH₂Cl₂ (6.0 ml) at 0 °C. After it had stirred for 1 hour, the solution was allowed to warm to room temperature and stirred for another 8 hours. The precipitate was filtered and washed with ethyl acetate (3 X 100 ml). The combined organic phases were washed with water and brine, and dried over anhydrous Na₂SO₄. Flash column chromatography on silica gel eluting with 80/1 hexane/ethyl acetate gave octyl cyclobut-1-enecarboxylate as a clear oil (0.604 g, 96%). To a -78 °C solution of this oil (0.211 g, 1.0 mmol) in CH₂Cl₂ (10 ml) was bubbled O₃/O₂ until the solution turned blue. The residual ozone was discharged by bubbling with O₂ and the reaction was warmed to room temperature and stirred for another 1 hour. Dimethyl sulfide (Me₂S, 0.11 ml, 1.5 mmol) was added to the mixture and it was stirred for another 2 hours. The CH₂Cl₂ was removed in vacuo and the crude product was dissolved in a solution of 2-methyl-2-butene (0.8 ml) in *t*-BuOH (3.0 ml). To this was added drop-wise a solution containing sodium chlorite (0.147 g, 1.3 mmol) and sodium dihydrogen phosphate monohydrate (0.179 g, 1.3 mmol) in H₂O (1.0 ml). The mixture was stirred at room temperature overnight, and then extracted with ethyl acetate (3 × 50 ml). The combined organic phases were washed with water and brine, and dried over anhydrous Na₂SO₄. Flash column chromatography on silica gel eluting with 5/1 hexane/ethyl acetate gave octyl α-KG, which became a pale solid when stored in the refrigerator (0.216 g, 84%).

### Synthesis of 5-octyl L-Glu ((S)-2-amino-5-(octyloxy)-5-oxopentanoic acid)

L-Glutamic acid (0.147 g, 1.0 mmol) and anhydrous sodium sulfate (0.1 g) was dissolved in octanol (2.0 ml), and then tetrafluoroboric acid-dimethyl ether complex (0.17 ml) was added. The suspended mixture was stirred at 21 °C overnight. Anhydrous THF (5 ml) was added to the mixture and it was filtered through a thick pad of activated charcoal. Anhydrous triethylamine (0.4 ml) was added to the clear filtrate to obtain a milky white slurry. Upon trituration with ethyl acetate (10 ml), the monoester monoacid precipitated. The precipitate was collected, washed with additional ethyl acetate (2X5 ml), and dried in vacuo to give the desired product 5-octyl L-Glu (0.249 g, 96%) as a white solid. ¹H NMR (500 MHz, Acetic acid-d₄): δ 4.12 (dd, *J* = 6.6, 6.6 Hz, 1H), 4.11 (t, *J* = 6.8 Hz, 2H), 2.64 (m, 2H), 2.26 (m, 2H), 1.64 (m, 2H), 1.30 (m, 10H), 0.89 (t, *J* = 7.0 Hz, 3H). ¹³C NMR (125 MHz, Acetic acid-d₄): 175.0, 174.3, 66.3, 55.0, 32.7, 30.9, 30.11, 30.08, 29.3, 26.7, 26.3, 23.4, 14.4.

### Synthesis of 5-octyl D-Glu ((R)-2-amino-5-(octyloxy)-5-oxopentanoic acid)

The synthesis of the opposite enantiomer, i.e., 5-octyl D-Glu, was carried out by the exact same procedure starting with D-glutamic acid. The spectroscopic data was identical to that of the enantiomeric compound.

### Synthesis of 5-octyl α-KG (5-(Octyloxy)-2,5-dioxopentanoic acid)

1-benzyl 5-octyl 2-oxopentanedioate: To a solution of 5-octyl L-Glu (0.249 g) in H₂O (6.0 ml) and acetic acid (2.0 ml) cooled to 0 °C was added slowly a solution of aqueous sodium nitrite (0.207 g, 3.0 mmol in 4 ml H₂O). The reaction mixture was allowed to warm slowly to room temperature and was stirred overnight. The mixture was concentrated. The resulting residue was dissolved in DMF (10 ml) and NaHCO₃ (0.42 g, 5.0 mmol) and benzyl bromide (0.242 ml, 2.0 mmol) were added to the mixture. The mixture was stirred at 21 °C overnight and then extracted with ethyl acetate (3 × 30 ml). The combined organic phase was washed with water and brine and dried over anhydrous MgSO₄. Flash column chromatography on silica gel eluting with 7/1 hexanes/ethyl acetate gave the mixed diester 1-benzyl 5-octyl (*S*)-2-hydroxypentanedioate as a colorless oil. To this oil dissolved in dichloromethane (10.0 ml), were added NaHCO₃ (0.42 g, 5.0 mmol) and Dess-Martin periodinane (0.509 g, 1.2 mmol) and the mixture was stirred at room temperature for 1 hour and then extracted with ethyl acetate (3 × 30 ml). The combined organic phase was washed with water and brine and dried over anhydrous MgSO₄. Flash column chromatography on silica gel eluting with 5/1 hexanes/ethyl acetate gave the desired 1-benzyl 5-octyl 2-oxopentanedioate (0.22 g, 66%) as a white solid. ¹H NMR (500 MHz, CDCl₃): 7.38 (m, 5H), 5.27 (s, 2H), 4.05 (t, *J* = 6.5 Hz, 2H), 3.14 (t, *J* = 6.5 Hz, 2H), 2.64 (t, *J* = 6.5 Hz, 2H), 1.59 (m, 2H), 1.28 (m, 10H), 0.87 (t, *J* = 7.0 Hz, 3H). ¹³C NMR (125 MHz, CDCl₃): 192.2, 171.9, 160.1, 134.3, 128.7, 128.6, 128.5, 67.9, 65.0, 34.2, 31.7, 29.07, 29.05, 28.4, 27.5, 25.7, 22.5, 14.0.

5-octyl α-KG (5-(Octyloxy)-2,5-dioxopentanoic acid): To a solution of 1-benzyl 5-octyl 2-oxopentanedioate (0.12 g, 0.344 mmol) in ethyl acetate (15 ml) was added 5% Pd/C (80 mg). Over the mixture was passed a stream of argon and then the argon was replaced with hydrogen gas and the mixture was stirred vigorously for 15 minutes. The mixture was filtered through a thick pad of Celite to give the desired product 5-octyl α-KG (0.088 g, 99%) as white solid. ¹H NMR (500 MHz, CDCl₃): 8.16 (br s, 1H), 4.06 (t, *J* = 6.5 Hz, 2H), 3.18 (t, *J* = 6.5 Hz, 2H), 2.69 (t, *J* = 6.0 Hz, 2H), 1.59 (m, 2H), 1.26 (m, 10H), 0.85 (t, *J* = 7.0 Hz, 3H). ¹³C NMR (125 MHz, CDCl₃): 193.8, 172.7, 160.5, 65.5, 33.0, 31.7, 29.08, 29.06, 28.4, 27.8, 25.8, 22.5, 14.0.

### Synthesis of 1-Octyl (S) 2-hydroxypentanedioate (Octyl (S)-2-HG)

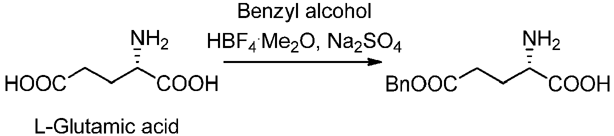

(*S*)-2-amino-5-(benzyloxy)-5-oxopentanoic acid: L-Glutamic acid (2.0 g, 13.6 mmol) and anhydrous sodium sulfate (2.0 g) was dissolved in benzyl alcohol (25 ml), and then tetrafluoroboric acid diethyl ether complex (3.7 ml, 27.2 mmol) was added. The suspended mixture was stirred at 21 °C overnight. Anhydrous THF (75 ml) was added to the mixture and it was filtered through a thick pad of activated charcoal. Anhydrous triethylamine (4.1 ml) was added to the clear filtrate to obtain a milky white slurry. Upon trituration with ethyl acetate (100 ml), the monoester monoacid precipitated. It was collected, washed with additional ethyl acetate (2 × 10 ml), and dried in vacuo to give the desired product (*S*)-2-amino-5-(benzyloxy)-5-oxopentanoic acid (3.07 g, 95%) as a white solid. ¹H NMR (500 MHz, Acetic acid-d₄): δ 7.41 - 7.25 (m, 5H), 5.14 (s, 2H), 4.12 (m, 1H), 2.75 - 2.60 (m, 2H), 2.27 (m, 2H). ¹³C NMR (125 MHz, Acetic acid-d₄): δ 174.6, 174.4, 136.9, 129.5, 129.2, 129.1, 67.7, 55.0, 30.9, 26.3.

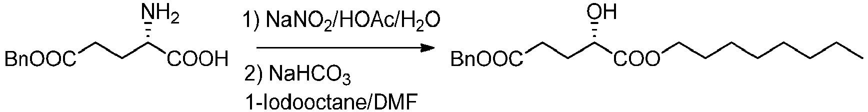

(S)-5-benzyl 1-octyl 2-hydroxypentanedioate: To a solution of (*S*)-2-amino-5-(benzyloxy)-5-oxopentanoic acid (1.187 g, 5.0 mmol) in H₂O (25 ml) and acetic acid (10 ml) cooled to 0 °C was added slowly a solution of aqueous sodium nitrite (1.07 g in 15 ml H₂O). The reaction mixture was allowed to warm slowly to room temperature and was stirred overnight. The mixture was concentrated. The resulting residue was dissolved in DMF (15 ml) and NaHCO₃ (1.26 g, 15 mmol) and 1-iodooctane (1.84 ml, 10 mmol) were added to the mixture. The mixture was stirred at 21 °C overnight and then extracted with ethyl acetate (3 × 50 ml). The combined organic phase was washed with water and brine and dried over anhydrous MgSO₄. Flash column chromatography on silica gel eluting with 7/1 hexanes/ethyl acetate gave the desired mixed diester (*S*)-5-benzyl 1-octyl 2-hydroxypentanedioate (0.785 g, 45%) as a colorless oil. ¹H NMR (500 MHz, CDCl₃): δ 7.37 - 7.28 (m, 5H), 5.12 (s, 2H), 4.26 - 4.19 (m, 1H), 4.16 (t, *J* = 6.8 Hz, 2H), 3.11 (d, *J* = 4.1 Hz, 1H), 2.61 - 2.46 (m, 2H), 2.26 - 2.14 (m, 1H), 1.95 (dtd, *J* = 14.2, 8.3, 6.1 Hz, 1H), 1.71 - 1.57 (m, 2H), 1.39 - 1.20 (m, 10H), 0.88 (t, *J* = 6.9 Hz, 3H). ¹³C NMR (125 MHz, CDCl₃): δ 174.6, 172.8, 135.8, 128.4, 128.1, 128.0, 69.3, 66.2, 65.8, 31.6, 29.6, 29.2, 29.0 (2C's), 28.4, 25.6, 22.5, 13.9.

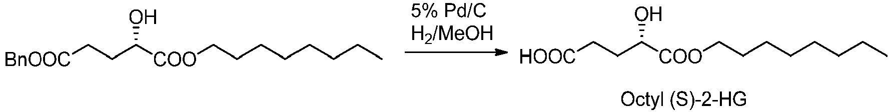

1-octyl (*S*) 2-hydroxypentanedioate (octyl (*S*)-2-hydroxyglutarate; octyl (S)-2-HG): To a solution of (*S*)-5-benzyl 1-octyl 2-hydroxypentanedioate (0.71 g, 2.0 mmol) in MeOH (50 ml) was added 5% Pd/C (80 mg). Over the mixture was passed argon condition and then the argon was replaced with hydrogen and the mixture was stirred vigorously for 1 hour. The mixture was filtered through a thick pad of Celite and the organic phase was evaporated. The residue was purified via flash column chromatography on silica gel eluting with 25/1 CH₂Cl₂/MeOH to give octyl (*S*)-2-HG (0.495 g, 48%) as white solid. ¹H NMR (500 MHz, CDCl₃): δ 4.23 (dd, *J* = 8.0, 4.2 Hz, 1H), 4.16 (t, *J* = 6.8 Hz, 2H), 2.60 - 2.42 (m, 2H), 2.15 (m, 1H), 1.92 (dtd, *J* = 14.2, 8.2, 6.1 Hz, 1H), 1.69 - 1.59 (m, 2H), 1.38 - 1.16 (m, 10H), 0.86 (t, *J* = 7.0 Hz, 3H). ¹³C NMR (125 MHz, CDCl₃): δ 178.8, 174.8, 69.3, 66.1, 31.7, 29.4, 29.1 (2C's), 28.9, 28.4, 25.7, 22.5, 14.0.

### Synthesis of 1-Octyl (R) 2-hydroxypentanedioate (Octyl (R)-2-HG)

The synthesis of the opposite enantiomer, i.e., octyl (*R*)-2-HG, was carried out by the exact same procedure starting with D-glutamic acid. The spectroscopic data was identical to that of the enantiomeric compounds.

### RNAi in C. elegans

RNAi in *C*. *elegans* was accomplished by feeding worms HT115(DE3) bacteria expressing target-gene double-stranded RNA (dsRNA) from the pL4440 vector. See Timmons & Fire, Nature 395, 854 (1998). dsRNA production was induced overnight on plates containing 1 mM IPTG. All RNAi feeding clones were obtained from the *C*. *elegans* ORF-RNAi Library (Thermo Scientific/Open Biosystems) unless otherwise stated. The *C*. *elegans* TOR (CeTOR) RNAi clone (Long et al. Curr Biol 12, 1448-1461 (2002)) was obtained from Joseph Avruch (MGH/Harvard). Efficient knockdown was confirmed by Western blotting of the corresponding protein or by qRT-PCR of the mRNA. The primer sequences used for qRT-PCR are as follows:
*atp-2* forward: TGACAACATTTTCCGTTTCACC (SEQ ID NO:1)
*atp-2* reverse: AAATAGCCTGGACGGATGTGAT (SEQ ID NO:2)
*let-363*/*CeTOR* forward: GATCCGAGACAAGATGAACGTG (SEQ ID NO:3)
*let-363*/*CeTOR* reverse: ACAATTTGGAACCCAACCAATC (SEQ ID NO:4)
*ogdh-1* forward: TGATTTGGACCGAGAATTCCTT (SEQ ID NO:5)
*ogdh-1* reverse: GGATCAGACGTTTGAACAGCAC (SEQ ID NO:6)

The RNAi knockdown of both *ogdh-1* and *atp-2* was validated by quantitative RT-PCR and *atp-2* knockdown was also validated by Western blotting. Transcripts of *ogdh-1* were reduced by 85%, and transcripts and protein levels of *atp-*2 were reduced by 52% and 83%, respectively, in larvae that were cultivated on bacteria that expressed the corresponding dsRNAs. In addition, RNAi of *atp-2* was found to be associated with delayed post-embryonic development and larval arrest, which is consistent with the phenotypes of *atp-2(ua2)* animals. Analysis by qRT-PCR indicated a modest but significant decrease by 26% in transcripts of *CeTOR* in larvae undergoing RNAi; moreover, molecular markers for autophagy were induced in these animals, and the lifespan of adults was extended, which is consistent with partial inactivation of the kinase.

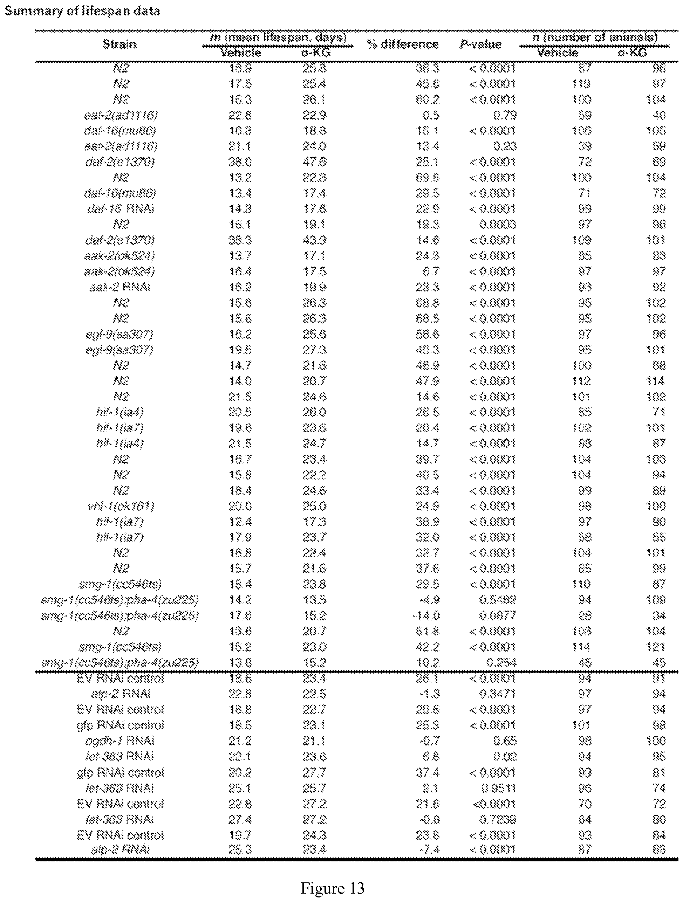

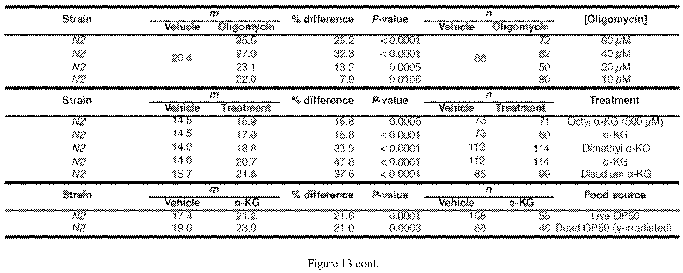

In lifespan experiments, RNAi was used to inactivate *atp-2*, *ogdh-1*, and *CeTOR* in mature animals in the presence or absence of exogenous α-KG. The concentration of α-KG used in these experiments (8 mM) was empirically determined to be most beneficial for wild-type animals (Figure 1, Panel c). This approach enabled the evaluation of the contribution of essential proteins and pathways to the longevity conferred by supplemental α-KG. Specifically, substantial, but not complete, inactivation of *atp-2* in adult animals that had completed embryonic and larval development was possible. As described herein, supplementation with 8 mM α-KG did not further extend (and in fact, in one occasion, even decreased) the lifespan of *atp-2(RNAi)* animals (Figure 13), thereby indicating that *atp-2* is involved. On the other hand, a complete inactivation of *atp-2* would be lethal, and thereby mask the benefit of ATP synthase inhibition by α-KG.

### Lifespan analysis

Lifespan assays were conducted at 20 °C on solid nematode growth media (NGM) using standard protocols and were replicated in at least two independent experiments. *C. elegans* were synchronized by performing either a timed egg lay (Sutphin & Kaeberlein, J Vis Exp (2009)) or an egg preparation (lysing about 100 gravid worms in 70 µl M9 buffer, 25 µl bleach (10% sodium hypochlorite solution), and 5 µl 10 N NaOH (Brenner Genetics 77, 71-94 (1974)). Young adult animals were picked onto NGM assay plates containing 1.5% dimethyl sulfoxide (DMSO; Sigma, D8418), 49.5 µM 5-fluoro-2'-deoxyuridine (Sutphin & Kaeberlein, J Vis Exp (2009)) (FUDR; Sigma, F0503), D-2-HG (Sigma, H8378), or L-2-HG (Sigma, 90790), and α-KG (Sigma, K1128) or vehicle control (H₂O). FUDR was included to prevent progeny production. Media containing α-KG were adjusted to pH 6.0 (the same pH as the control plates) by the addition of NaOH. All compounds were mixed into the NGM media after autoclaving and before solidification of the media. Assay plates were seeded with OP50 (or a designated RNAi feeding clone, see below). Worms were moved to new assay plates every 4 days (to ensure sufficient food was present at all times and to reduce the risk of mold contamination). To assess the survival of the worms, the animals were prodded with a platinum wire every 2-3 days, and those that failed to respond were scored as dead. For analysis concerning mutant strains, the corresponding parent strain was used as a control in the same experiment.

For lifespan experiments involving RNAi, the plates also contained 1 mM isopropyl β-D-1-thiogalactopyranoside (IPTG; Acros, CAS 367-93-1) and 50 µg/ml ampicillin (Fisher, BP1760-25). RNAi was accomplished by feeding N2 worms HT115(DE3) bacteria expressing target-gene dsRNA from pL4440 (Timmons & Fire, Nature 395, 854 (1998)); control RNAi was done in parallel for every experiment by feeding N2 worms HT115(DE3) bacteria expressing either GFP dsRNA or empty vector (which gave identical lifespan results).

Lifespan experiments with oligomycin (Cell Signaling Technology, 9996) were performed as described for α-KG (NGM plates with 1.5% DMSO and 49.5 µM FUDR; N2 worms; OP50 bacteria).

For lifespan experiments concerning *smg-1(cc546ts);pha-4(zu225) and smg-1(cc546ts)* (Timmons & Fire, Nature 395, 854 (1998); and Gaudet & Mango, Science 295, 821-825 (2002)), the strains were grown from egg to L4 stage at 24 °C, which inactivates the *smg-1* temperature-sensitive allele, preventing mRNA surveillance-mediated degradation of the *pha-4(zu225)* mRNA, which contains a premature stop codon, and thus produces a truncated but fully functional PHA-4 transcription factor (Gaudet & Mango, Science 295, 821-825 (2002)). Then at the L4 stage the temperature was shifted to 20 °C, which restores *smg-1* function and thereby results in the degradation of *pha-4(zu225)* mRNA. Treatment with α-KG began at the L4 stage.

Lifespan data is provided in Figure 13, including sample sizes. The sample size was chosen on the basis of standards done in the field in published manuscripts. No statistical method was used to predetermine the sample size. Animals were assigned randomly to the experimental groups. Worms that ruptured, bagged (e.g., exhibited internal progeny hatching), or crawled off the plates were censored. Lifespan data were analyzed using GraphPad Prism; *P*-values were calculated using the log-rank (Mantel-Cox) test.

### Food Preference Assay

A protocol adapted from Abada et al. (Mol Cells 28, 209-213 (2009)) was performed as follows. A 10 cm NGM plate was seeded with two spots of OP50 as shown in Figure 5, Panel e. After letting the OP50 lawns to dry over 2 days at room temperature, vehicle (H₂O) or α-KG (8 mM) was added to the top of the lawn and allowed to dry over 2 days at room temperature. About 50-100 synchronized adult day 1 worms were placed onto the center of the plate and their preference for either bacterial lawn was recorded after 3 hours at room temperature.

### Target Identification Using Drug Affinity Responsive Target Stability (DARTS)

Target identification using DARTS was conducted in accordance with methods know in the art. See e.g., Lomenick, et al. PNAS USA 106, 21984-21989 (2009). For unbiased target ID (Figure 2, Panel a), human Jurkat cells were lysed using M-PER (Thermo Scientific, 78501) with the addition of protease inhibitors (Roche, 11836153001) and phosphatase inhibitors (Lomenick, et al. Curr Protoc Chem Biol 3, 163-180 (2011)). TNC buffer (50 mM Tris-HCl pH 8.0, 50 mM NaCl, 10 mM CaCl₂) was added to the lysate and protein concentration was then determined using the BCA Protein Assay kit (Pierce, 23227). Cell lysates were incubated with either vehicle (H₂O) or α-KG for 1 hour on ice followed by an additional 20 minutes at room temperature. Digestion was performed using Pronase (Roche, 10165921001) at room temperature for 30 minutes and stopped using excess protease inhibitors with immediate transfer to ice. The resulting digests were separated by SDS-PAGE and visualized using SYPRO Ruby Protein Gel Stain (Invitrogen, S12000). The band with increased staining from the α-KG lane (corresponding to potential protein targets that are protected from proteolysis by the binding of α-KG) and the matching area of the control lane were excised, in-gel trypsin digested, and subjected to LC-MS/MS analysis as described (Lomenick, et al. PNAS USA 106, 21984-21989 (2009); and Lomenick, et al. ACS Chem Biol 6, 34-46 (2011)). Mass spectrometry results were searched against the human Swissprot database (release 57.15) using Mascot version 2.3.0, with all peptides meeting a significance threshold of 0.05.

For target verification by DARTS-Western blotting (Figure 2, Panel b), HeLa cells were lysed in M-PER buffer (Thermo Scientific, 78501) with the addition of protease inhibitors (Roche, 11836153001) and phosphatase inhibitors (50 mM NaF, 10 mM β-glycerophosphate, 5 mM sodium pyrophosphate, 2 mM Na₃VO₄). Chilled TNC buffer (50 mM Tris-HCl pH 8.0, 50 mM NaCl, 10 mM CaCl₂) was added to the protein lysate, and protein concentration of the lysate was measured by the BCA Protein Assay kit (Pierce, 23227). The protein lysate was then incubated with vehicle control (H₂O) or varying concentrations of α-KG for 3 hours at room temperature with shaking at 600 rpm in an Eppendorf Thermomixer. Pronase (Roche, 10165921001) digestions were performed for 20 minutes at room temperature, and stopped by adding SDS loading buffer and immediately heating at 70 °C for 10 minutes. Samples were subjected to SDS-PAGE on 4-12% Bis-Tris gradient gel (Invitrogen, NP0322BOX) and Western blotted for ATP synthase subunits ATP5B (Sigma, AV48185), ATP5O (Abcam, ab91400), and ATP5A (Abcam, ab110273). Binding between α-KG and PHD-2/Egln1 (D31E11) Rabbit mAb (Cell Signaling Technology, 4835), for which α-KG is a co-substrate (Stubbs, et al. J Med Chem 52, 2799-2805 (2009)), was confirmed by DARTS. GAPDH (Ambion, AM4300) was used as a negative control.

For DARTS using *C*. *elegans* (Figure 6, Panel a), wild-type animals of various ages were grown on NGM/OP50 plates, washed 4 times with M9 buffer, and immediately placed in the -80 °C freezer. Animals were lysed in HEPES buffer (40 mM HEPES pH 8.0, 120 mM NaCl, 10% glycerol, 0.5 % Triton X-100, 10 mM β-glycerophosphate, 50 mM NaF, 0.2 mM Na₃VO₄, protease inhibitors (Roche, 11836153001)) using Lysing Matrix C tubes (MP Biomedicals, 6912-100) and the FastPrep-24 (MP Biomedicals) high-speed benchtop homogenizer in the 4 °C room (disrupt worms for 20 seconds at 6.5 m/s, rest on ice for 1 minute; repeat twice). Lysed animals were centrifuged at 14,000 rpm for 10 minutes at 4 °C to pellet worm debris, and supernatant was collected for DARTS. Protein concentration was determined by BCA Protein Assay kit (Pierce, 23223). A worm lysate concentration of 1.13 µg/µl was used for the DARTS experiment. All steps were performed on ice or at 4 °C to help prevent premature protein degradation. TNC buffer (50 mM Tris-HCl pH 8.0, 50 mM NaCl, 10 mM CaCl₂) was added to the worm lysates. Worm lysates were incubated with vehicle control (H₂O) or α-KG for 1 hour on ice and then 50 minutes at room temperature. Pronase (Roche, 10165921001) digestions were performed for 30 minutes at room temperature and stopped by adding SDS loading buffer and heating at 70 °C for 10 minutes. Samples were then subjected to SDS-PAGE on NuPAGE Novex 4-12% Bis-Tris gradient gels (Invitrogen, NP0322BOX), and Western blotting was carried out with an antibody against ATP5B (Sigma, AV48185) that also recognizes ATP-2.

For 2-HG experiments, DARTS was performed as described above. Briefly, U87 cells were lysed in M-PER buffer (Thermo Scientific, 78501) with the addition of protease (Roche, 11836153001) and phosphatase (50 mM NaF, 2 mM Na₃VO₄) inhibitors. Chilled TNC buffer (50 mM Tris-HCl pH 8.0, 50 mM NaCl, 10 mM CaCl₂) was added to the lysate, and protein concentration of the solution was measured on an aliquot by the BCA Protein Assay kit (Pierce, 23227). The remaining lysate was then incubated with vehicle control (H₂O) or varying concentrations of 2-HG or α-KG for 0.5 hours at room temperature. The samples were then subjected to Pronase digestions (Roche, 10165921001, 5 minutes at room temperature) that were stopped by addition of SDS loading buffer and immediate heating (95 °C, 5 minutes). Samples were subjected to SDS-PAGE on 4-12% Bis-Tris gradient gel (Invitrogen, NP0322BOX), and Western blotting was carried out with antibodies against ATP5B (Sigma, AV48185) or GAPDH (Santa Cruz, SC25778).

### Complex V Activity Assay

Complex V activity was assayed using the MitoTox OXPHOS Complex V Activity Kit (Abcam, ab109907). Vehicle (H₂O) or α-KG was mixed with the enzyme prior to the addition of phospholipids. In experiments using octyl α-KG, vehicle (1% DMSO) or octyl α-KG was added with the phospholipids. Relative Complex V activity was compared to vehicle. Oligomycin (Sigma, O4876) was used as a positive control for the assay.

### Isolation of Mitochondria from Mouse Liver

Animal studies were performed under approved UCLA animal research protocols. Mitochondria from 3-month-old C57BL/6 mice were isolated as described (Rogers, et al. PLoS One 6, e21746 (2011)). Briefly, livers were extracted, minced at 4 °C in MSHE+BSA (70 mM sucrose, 210 mM mannitol, 5 mM HEPES, 1 mM EGTA, and 0.5% fatty acid free BSA, pH 7.2), and rinsed several times to remove blood. All subsequent steps were performed on ice or at 4 °C. The tissue was disrupted in 10 volumes of MSHE+BSA with a glass Dounce homogenizer (5-6 strokes) and the homogenate was centrifuged at 800 × g for 10 minutes to remove tissue debris and nuclei. The supernatant was decanted through a cell strainer and centrifuged at 8,000 × g for 10 minutes. The dark mitochondrial pellet was resuspended in MSHE+BSA and recentrifuged at 8,000 × g for 10 minutes. The final mitochondrial pellets were used for various assays as described below.

### Submitochondrial Particle (SMP) ATPase Assay

ATP hydrolysis by ATP synthase was measured using submitochondrial particles (see Alberts, B. Molecular Biology of the Cell. 3rd edn, (Garland Pub., 1994) and references therein). Mitochondria were isolated from mouse liver as described above. The final mitochondrial pellet was resuspended in buffer A (250 mM sucrose, 10 mM Tris-HCl, 1 mM ATP, 5 mM MgCl₂, and 0.1 mM EGTA, pH 7.4) at 10 µg/µl, subjected to sonication on ice (Fisher Scientific Model 550 Sonic Dismembrator; medium power, alternating between 10 second intervals of sonication and resting on ice for a total of 60 seconds of sonication), and then centrifuged at 18,000 × g for 10 minutes at 4 °C. The supernatant was collected and centrifuged at 100,000 × g for 45 minutes at 4 °C. The final pellet (submitochondrial particles) was resuspended in buffer B (250 mM sucrose, 10 mM Tris-HCl, and 0.02 mM EGTA, pH 7.4).

The SMP ATPase activity was assayed using the Complex V Activity Buffer as above. The production of ADP is coupled to the oxidation of NADH to NAD⁺ through pyruvate kinase and lactate dehydrogenase. The addition of α-KG (up to 10 mM) did not affect the activity of pyruvate kinase or lactate dehydrogenase when external ADP was added. The absorbance decrease of NADH at 340 nm correlates to ATPase activity. SMPs (2.18 ng/µl) were incubated with vehicle or α-KG for 90 minutes at room temperature prior to the addition of activity buffer, and then the absorbance decrease of NADH at 340 nm was measured every 1 minute for 1 hour. Oligomycin (Cell Signaling Technology, 9996) was used as a positive control for the assay.

### Assay for ATP Levels

Normal human diploid fibroblast WI-38 (ATCC, CCL-75) cells were seeded in 96-well plates at 2 × 10⁴ cells per well. Cells were treated with either DMSO (vehicle control) or octyl α-KG at varying concentrations for 2 hours in triplicate. ATP levels were measured using the CellTiter-Glo luminescent ATP assay (Promega, G7572); luminescence was read using Analyst HT (Molecular Devices). In parallel, identically treated cells were lysed in M-PER (Thermo Scientific, 78501) to obtain protein concentration by BCA Protein Assay kit (Pierce, 23223). ATP levels were normalized to protein content. Statistical analysis was performed using GraphPad Prism (unpaired t-test).

Assay for ATP levels in *C*. *elegans.* Synchronized day 1 adult wild-type *C*. *elegans* were placed on NGM plates containing either vehicle or 8 mM α-KG. On day 2 and 8 of adulthood, 9 replicates and 4 replicates, respectively, of about 100 worms were collected from α-KG or vehicle control plates, washed 4 times in M9 buffer, and frozen in -80 °C. Animals were lysed using Lysing Matrix C tubes (MP Biomedicals, 6912-100) and the FastPrep-24 (MP Biomedicals) high-speed benchtop homogenizer (disrupt worms for 20 seconds at 6.5 m/s, rest on ice for 1 minute; repeat twice). Lysed animals were centrifuged at 14,000 rpm for 10 minutes at 4 °C to pellet worm debris, and supernatant was saved for ATP quantitation using the Kinase-Glo Luminescent Kinase Assay Platform (Promega, V6713) according to manufacturer's instructions. Assay was performed in white opaque 96 well tissue culture plates (Falcon, 353296), and luminescence was measured using Analyst HT (Molecular Devices). ATP levels were normalized to number of worms. Statistical analysis was performed using Microsoft Excel (t-test, two-tailed, two-sample unequal variance).

For 2-HG experiments, U87 cells were seeded in 96-well plates at 2 × 10⁴ cells per well and treated with indicated compound for 2 hours in triplicate. ATP levels were measured using the CellTiter-Glo luminescent ATP assay (Promega, G7572); luminescence was read using Analyst HT (Molecular Devices). To confirm that the number of cells was consistent between treatments, cell lysates were further subjected to dsDNA staining using QuantiFluor dsDNA system (Promega). Statistical analysis was performed using GraphPad Prism (unpaired *t*-test).

### Measurement of Oxygen Consumption Rates (OCR) and Extracellular Acidification Rates (ECAR)

OCR measurements were made using a Seahorse XF-24 analyzer (Seahorse Bioscience) (Wu, et al. Am J Physiol Cell Physiol 292, C125-136 (2007)). Cells were seeded in Seahorse XF-24 cell culture microplates at 50,000 cells/well in DMEM media supplemented with 10% FBS and 10 mM glucose, and incubated at 37 °C and 5% CO₂ for overnight. Treatment with octyl α-KG or DMSO (vehicle control) was for 1 hour. Cells were washed in unbuffered DMEM medium (pH 7.4, 10 mM glucose) just prior to measurement, and maintained in this buffer with indicated concentrations of octyl α-KG. Oxygen consumption rates were measured 3 times under basal conditions and normalized to protein concentration per well. Statistical analysis was performed using GraphPad Prism.

Measurement of oxygen consumption rates (OCR) in living *C*. *elegans* was performed using a protocol adapted from Yamamoto, et al. (Cell 147, 827-839 (2011)) and Pathare, et al. (PLoS Genet 8, e1002645 (2012)). Wild-type day 1 adult N2 worms were placed on NGM plates containing 8 mM α-KG or H₂O (vehicle control) seeded with OP50 or HT115 *E. coli.* OCR was assessed on day 2 of adulthood. On day 2 of adulthood, worms were collected and washed 4 times with M9 to rid the samples of bacteria (we further verified that α-KG does not affect oxygen consumption of the bacteria - therefore, even if there were any leftover bacteria after the washes, the changes in OCR observed would still be worm-specific), and then the animals were seeded in quadruplicates in Seahorse XF-24 cell culture microplates (Seahorse Bioscience, V7-PS) in 200 µl M9 at about 200 worms per well. Oxygen consumption rates were measured 7 times under basal conditions and normalized to the number of worms counted per well. The experiment was repeated twice. Statistical analysis was performed using Microsoft Excel (t-test, two-tailed, two-sample unequal variance).

For 2-HG experiments, OCR and ECAR measurements were made using a Seahorse XF-24 analyzer (Seahorse Bioscience) (Wu, et al. Am J Physiol Cell Physiol 292, C125-136 (2007)). U87 cells were seeded in Seahorse XF-24 cell culture microplates at 50,000 cells per well in DMEM supplemented with 10% FBS and either 10 mM glucose or 10 mM galactose, and incubated O/N at 37 °C in 5% CO₂. Treatment with octyl α-KG, octyl (R)-2-HG, octyl (S)-2-HG, or DMSO (vehicle control) was for 1 hour. Cells were washed in unbuffered DMEM (pH 7.4, 10 mM glucose) immediately prior to measurement, and maintained in this buffer with indicated concentrations of compound. OCR or ECAR were measured 3 times under basal conditions and normalized to protein concentration per well. Statistical analysis was performed using GraphPad Prism (unpaired *t*-test, two-tailed, two-sample unequal variance).

### Measurement of Mitochondrial Respiratory Control Ratio (RCR)

Mitochondrial RCR was analyzed using isolated mouse liver mitochondria (see Brand, et al. Biochem J 435, 297-312 (2011) and references therein). Mitochondria were isolated from mouse liver as described above. The final mitochondrial pellet was resuspended in 30 µl of MAS buffer (70 mM sucrose, 220 mM mannitol, 10 mM KH₂PO₄, 5 mM MgCl₂, 2 mM HEPES, 1 mM EGTA, and 0.2% fatty acid free BSA, pH 7.2).

Isolated mitochondrial respiration was measured by running coupling and electron flow assays as described (Rogers, et al. PLoS One 6, e21746 (2011)). For the coupling assay, 20 µg of mitochondria in complete MAS buffer (MAS buffer supplemented with 10 mM succinate and 2 µM rotenone) were seeded into a XF24 Seahorse plate by centrifugation at 2,000 × g for 20 minutes at 4 °C. Just before the assay, the mitochondria were supplemented with complete MAS buffer for a total of 500 µl (with 1% DMSO, octanol, octyl α-KG, or octyl 2-HG), and warmed at 37 °C for 30 minutes before starting the oxygen consumption rate measurements. Mitochondrial respiration begins in a coupled State 2; State 3 is initiated by 2 mM ADP; State 4o (oligomycin-insensitive, i.e., Complex V-independent) is induced by 2.5 µM oligomycin and State 3u (FCCP uncoupled maximal respiratory capacity) by 4 µM FCCP. Finally, 1.5 µg/ml antimycin A was injected at the end of the assay. The State 3/State 4o ratio gives the respiratory control ratio (RCR).

For the electron flow assay, the MAS buffer was supplemented with 10 mM sodium pyruvate (Complex I substrate), 2 mM malate (Complex II inhibitor), and 4 µM FCCP, and the mitochondria are seeded the same way as described for the coupling assay. After basal readings, the sequential injections were as follows: 2 µM rotenone (Complex I inhibitor), 10 mM succinate (Complex II substrate), 4 µM antimycin A (Complex III inhibitor), and 10 mM/100 µM ascorbate/tetramethylphenylenediamine (Complex IV substrate).

### ATP Synthase Enzyme Inhibition Kinetics

ATP synthesis enzyme inhibition kinetic analysis was performed using isolated mitochondria. Mitochondria were isolated from mouse liver as described above. The final mitochondrial pellet was resuspended in MAS buffer supplemented with 5 mM sodium ascorbate (Sigma, A7631) and 5 mM TMPD (Sigma, T7394).

The reaction was carried out in MAS buffer containing 5 mM sodium ascorbate, 5 mM TMPD, luciferase reagent (Roche, 11699695001), octanol or octyl α-KG, variable amounts of ADP (Sigma, A2754), and 3.75 ng/µl mitochondria. ATP synthesis was monitored by the increase in luminescence over time by a luminometer (Analyst HT, Molecular Devices). ATP synthase-independent ATP formation, derived from the oligomycin-insensitive luminescence, was subtracted as background. The initial velocity of ATP synthesis was calculated from the slope of the first 3 minutes of the reaction, before the velocity begins to decrease. Enzyme inhibition kinetics was analyzed by nonlinear regression least squares fit using GraphPad Prism.

### Assay for Mammalian TOR (mTOR) Pathway Activity

mTOR pathway activity in cells treated with octyl α-KG, 2-HG, or oligomycin was determined by the levels of phosphorylation of known mTOR substrates, including S6K (T389), 4E-BP1 (S65), AKT (S473), and ULK1 (S757) (Pullen & Thomas, FEBS Lett 410, 78-82 (1997); Burnett, et al. PNAS USA 95, 1432-1437 (1998); Gingras, et al. Genes Dev 15, 2852-2864 (2001); Sarbassov, et al. Science 307, 1098-1101 (2005); and Kim, et al. Nat Cell Biol 13, 132-141 (2011)). Specific antibodies used: P-S6K T389 (Cell Signaling Technology, 9234), S6K (Cell Signaling Technology, 9202S), P-4E-BP1 S65 (Cell Signaling Technology, 9451S), 4E-BP1 (Cell Signaling Technology, 9452S), P-AKT S473 (Cell Signaling Technology, 4060S), AKT (Cell Signaling Technology, 4691S), P-ULK1 S757 (Cell Signaling Technology, 6888), ULK1 (Cell Signaling Technology, 4773S), and GAPDH (Santa Cruz Biotechnology, 25778).

### Assay for Autophagy

DA2123 animals carrying an integrated GFP::LGG-1 translational fusion gene (Kang, et al. Genes Dev 21, 2161-2171 (2007); Hansen, et al. PLoS Genet 4, e24 (2008); and Alberti, et al. Autophagy 6, 622-633 (2010)), were used to quantify levels of autophagy. To obtain a synchronized population of DA2123, an egg preparation of gravid adults was prepared (by lysing about 100 gravid worms in 70 µl M9 buffer, 25 µl bleach and 5 µl 10 N NaOH), and the eggs were allowed to hatch overnight in M9 causing starvation induced L1 diapause. L1 larvae were deposited onto NGM treatment plates containing vehicle, 8 mM α-KG, or 40 µM oligomycin, and seeded with either *E*. *coli* OP50, HT115(DE3) with an empty vector, or HT115(DE3) expressing dsRNAs targeting *atp-2*, *CeTOR*/*let-363*, or *ogdh-1* as indicated. When the majority of animals in a given sample first reached the mid L3 stage, individual L3 larvae were mounted onto microscope slides and anesthetized with 1.6 mM levamisole (Sigma, 31742). Nematodes were observed using an Axiovert 200M Zeiss confocal microscope with a LSM5 Pascal laser, and images were captured using the LSM Image Examiner (Zeiss). For each specimen, GFP::LGG-1 puncta (autophagosomes) in the epidermis, including the lateral seam cells and Hyp7, were counted in three separate regions of 140.97 µm² using *analyze particles* in ImageJ (Schneider, et al. Nat Methods 9, 671-675 (2012)). Measurements were made blind to both the genotype and supplement. Statistical analysis was performed using Microsoft Excel (*t*-test, two-tailed, two-sample unequal variance).

Assay for autophagy in mammalian cells. HEK-293 cells were seeded in 6-well plates at 2.5 × 10⁵ cells/well in DMEM media supplemented with 10% FBS and 10 mM glucose, and incubated overnight before treatment with either octanol (vehicle control) or octyl α-KG for 72 hours. Cells were lysed in M-PER buffer with protease and phosphatase inhibitors. Lysates were subjected to SDS-PAGE on a 4-12% Bis-Tris gradient gel with MES running buffer and Western blotted for LC3 (Novus, NB100-2220). LC3 is the mammalian homolog of worm LGG-1, and conversion of the soluble LC3-I to the lipidated LC3-II is activated in autophagy, e.g., upon starvation (Kabeya, et al. EMBO J 19, 5720-5728 (2000)).

### Pharyngeal Pumping Rates of C. elegans Treated with 8 mM α-KG

The pharyngeal pumping rates of 20 wild-type N2 worms per condition were assessed. Pharyngeal contractions were recorded for 1 minute using a Zeiss M2BioDiscovery microscope and an attached Sony NDR-XR500V video camera at 12-fold optical zoom. The resulting videos were played back at 0.3x speed using MPlayerX and pharyngeal pumps were counted. Statistical analysis was performed using Microsoft Excel (t-test, two-tailed, two-sample unequal variance).

### Assay for α-KG Levels in C. elegans

Synchronized adult worms were collected from plates with vehicle (H₂O) or 8 mM α-KG, washed 3 times with M9 buffer, and flash frozen. Worms were lysed in M9 using Lysing Matrix C tubes (MP Biomedicals, 6912-100) and the FastPrep-24 (MP Biomedicals) high-speed benchtop homogenizer in the 4 °C room (disrupt worms for 20 seconds at 6.5 m/s, rest on ice for 1 minute; repeat three times). Lysed animals were centrifuged at 14,000 rpm for 10 minutes at 4 °C to pellet worm debris, and the supernatant was saved. The protein concentration of the supernatant was determined by the BCA Protein Assay kit (Pierce, 23223); there was no difference in protein level per worm in α-KG treated and vehicle treated animals (data not shown). α-KG content was assessed as described previously (MacKenzie, et al. Mol Cell Biol 27, 3282-3289 (2007)) with modifications. Worm lysates were incubated at 37 °C in 100 mM KH₂PO₄ (pH 7.2), 10 mM NH₄Cl, 5 mM MgCl₂, and 0.3 mM NADH for 10 minutes. Glutamate dehydrogenase (Sigma, G2501) was then added to reach a final concentration of 1.83 units/ml. Under these conditions glutamate dehydrogenase uses α-KG and NADH to make glutamate. The absorbance decrease was monitored at 340 nm. The intracellular level of α-KG was determined from the absorbance decrease in NADH. The approximate molarity of α-KG present inside the animals was estimated using average protein content (about 245 ng/worm, from BCA assay) and volume (about 3 nL for adult worms 1.1 mm in length and 60 µm in diameter (WorldWideWeb.wormatlasDOTORG/hermaphrodite/introduction/Introframeset.Hyper TextMarkupLanguage, wherein "WorldWideWeb" is "www", "DOTORG" is ".org", and "HyperTextMarkupLanguage" is "html")).

For quantitative analysis of α-KG in worms using UHPLC-ESI/MS/MS, synchronized day 1 adult worms were placed on vehicle plates with or without bacteria for 24 h, and then collected and lysed in the same manner as above. α-KG analysis by LC/MS/MS was carried out on an Agilent 1290 Infinity UHPLC system and 6460 Triple Quadrupole mass spectrometer (Agilent Technologies) using an electrospray ionization (ESI) source with Agilent Jet Stream technology. Data were acquired with Agilent MassHunter Data Acquisition software version B.06.00, and processed for precursor and product ions selection with MassHunter Qualitative Analysis software version B.06.00 and for calibration and quantification with MassHunter Quantitative Analysis for QQQ software version B.06.00.

For UHPLC, 3 µl calibration standards and samples were injected onto the UHPLC system including a G4220A binary pump with a built-in vacuum degasser and a thermostatted G4226A high performance autosampler. An ACQUITY UPLC BEH Amide analytical column (2.1 × 50 mm, 1.7 µm) and a VanGuard BEH Amide Pre-column (2.1 × 5 mm, 1.7 µm) from Waters Corporation were used at the flow rate of 0.6 ml/min using 50/50/0.04 acetonitrile/water/ammonium hydroxide with 10 mM ammonium acetate as mobile phase A and 95/5/0.04 acetonitrile/water/ammonium hydroxide with 10 mM ammonium acetate as mobile phase B. The column was maintained at room temperature. The following gradient was applied: 0-0.41 min: 100% B isocratic; 0.41-5.30 min: 100-30% B; 5.3-5.35 min: 30-0% B; 5.35-7.35 min: 0% B isocratic; 7.35-7.55 min: 0-100%B; 7.55-9.55 min: 100% B isocratic.

For the MS detection, the ESI mass spectra data were recorded on a negative ionization mode by MRM. MRM transitions of α-KG and its ISTD ¹³C₄-α-KG (Cambridge Isotope Laboratories) were determined using a 1-min 37% B isocratic UHPLC method through the column at flow rate of 0.6 ml/min. The precursor ion of [M-H]⁻ and the product ion of [M-CO₂-H]⁻ were observed to have the highest signal to noise ratios. The precursor and product ions are respectively 145.0 and 100.9 for AKG, and 149.0 and 104.9 for ISTD ¹³C₄-α-KG. Nitrogen was used as the drying, sheath, and collision gas. All the source and analyzer parameters were optimized using Agilent MassHunter Source and iFunnel Optimizer and Optimizer software respectively. The source parameters are as follows: drying gas temperature 120 °C, drying gas flow 13 L/min, nebulizer pressure 55 psi, sheath gas temperature 400 °C, sheath gas flow 12 L/min, capillary voltage 2000 V, and nozzle voltage 0 V. The analyzer parameters are as follows: fragmentor voltage 55 V, collision energy 2 V, and cell accelerator voltage 1 V. The UHPLC eluants before 1 minute and after 5.3 minutes were diverted to waste.

### Membrane Permeable Esters of α-KG

Octyl α-KG, a membrane-permeable ester of α-KG (MacKenzie, et al. Mol Cell Biol 27, 3282-3289 (2007); Zhao, et al. Science 324, 261-265 (2009); Xu, et al. Cancer Cell 19, 17-30 (2011); and Jin, et al. Cancer Res 73, 496-501 (2013)), was used to deliver α-KG across lipid membranes in experiments using cells and mitochondria. Upon hydrolysis by cellular esterases, octyl α-KG yields α-KG and the byproduct octanol. It was found that, whereas octanol control has no effect (Figure 6, Panels e-f and Figure 10, Panel a), α-KG alone can bind and inhibit ATP synthase (Figure 2, Panels a-b, Figure 6, Panels a-b, and data not shown), decrease ATP and OCR (Figure 2, Panel e, and Figure 2, Panel g), induce autophagy (Figure 4, Panel b), and increase *C*. *elegans* lifespan (Figure 1, Figure 3, Figure 5, Figure 9, and Figure 13). The existence and activity of esterases in the mitochondrial and cell culture experiments were confirmed using calcein AM (C1430, Molecular Probes), an esterase substrate, which fluoresces upon hydrolysis, and also by mass spectrometry (data not shown). The hydrolysis by esterases explains why distinct esters of α-KG, such as 1-octyl α-KG, 5-octyl α-KG, and dimethyl α-KG, have similar effects to α-KG (Figure 6, Panels g-h, and Figure 13).

### Cell Growth and Viability Assays

Cells were seeded in 12-well plates and after overnight incubation were treated with indicated concentrations of each compound. After harvesting, cells were stained by Acridine Orange (AO) and DAPI. Cell number and viability were measured based on AO and DAPI fluorescence measured by NC3000 (Chemometec) following the manufacturer's instructions.

### Metabolic Profile Analysis

Cells were cultured for 24 hours, rinsed with PBS, and medium containing [1,2-¹³C]glucose (1 g/L) added. After 24 hours culture, cells were rinsed with ice-cold 150 mM NH₄AcO (pH 7.3) followed by addition of 400 ml cold methanol and 400 ml cold water. Cells were scraped off, transferred to an Eppendorf tube, and 10 nmol norvaline as well as 400 ml chloroform added to each sample. For the metabolite extraction, samples were vortexed for 5 minutes on ice, spun down, and the aqueous layer transferred into a glass vial and dried. Metabolites were resuspended in 70% ACN, and 5 ml sample loaded onto a Phenomenex Luna 3u NH2 100A (150 × 2.0 mm) column. The chromatographic separation was performed on an UltiMate 300RSLC (Thermo Scientific) with mobile phases A (5 mM NH₄AcO, pH 9.9) and B (ACN) and a flow rate of 300 ml / minutes. The gradient ran from 15% A to 95% A over 18 minutes, 9 minutes isocratic at 95% A, and re-equilibration for 7 minutes. Metabolite detection was achieved with a Thermo Scientific Q Exactive mass spectrometer run in polarity switching mode (+3.0 kV / -2.25 kV). TraceFinder 3.1 (Thermo Scientific) was used to quantify metabolites as area under the curve using retention time and accurate mass measurements (≤ 3 ppm). Relative amounts of metabolites were calculated by summing up all isotopomers of a given metabolite.

### Assay for ADP Import

Freshly prepared mice liver mitochondria were suspended at 1 µg/µl in medium consisting of 220 mM mannitol, 70 mM sucrose, 2 mM HEPES, 2.74 µM antimycin A, 5 µM rotenone, 1 mM

EGTA, and 10 mM potassium phosphate buffer, pH 7.4. The mitochondria suspension was incubated with designated drug for 30 minutes in 37 °C. After incubation, the suspension was transferred to ice for 10 minutes incubation. Afterwards, 100 µM [³H]ADP (specific radioactivity, 185 kBq/pmol) was added, and the mixture was immediately vortexed and incubated for 20 seconds on ice. The reaction was terminated by addition of 10 µM carboxyatractyloside, and the mixture was centrifuged at 10,000 g for 10 minutes at 4 °C. After centrifuge, the supernatant was collected for reading and the pellet was washed twice with the same medium supplemented with 10 µM carboxyatractyloside. After washing, the pellet was lysed by the addition of 0.2 ml of 1% SDS. The radioactivity of the lysate and supernatant was determined by TRI-CARB 2300 TR liquid scintillation analyzer. The ADP-ATP translocation rate was determined by the ratio of the pellet versus the sum reading of the pellet and supernatant.

### Statistical Analyses

All experiments were repeated at least two times with identical or similar results. Data represent biological replicates. Appropriate statistical tests were used for every figure. Data meet the assumptions of the statistical tests described for each figure. Mean ± s.d. is plotted in all figures unless stated otherwise.

### RESULTS

As shown in Figure 1, α-KG extends the adult lifespan of C. *elegans.* Panel a shows that α-KG extends the lifespan of adult worms in the metabolite longevity screen. 8 mM was used for all metabolites. Panel b shows the structure of α-KG. Panel c shows the dose response of α-KG in longevity. Panels d-e show that α-KG extends the lifespan of worms fed ampicillin-arrested (Panel d) or γ-irradiation-killed (Panel e) bacteria (*P* < 0.0001). Panel f shows that α-KG does not further extend the lifespan of *ogdh-1 (RNAi)* worms (P = 0.65).

As shown in Figure 2, α-KG binds and inhibits ATP synthase. Panel a is a Sypro Ruby-stained gel identifying ATP5B as an α-KG-binding protein using DARTS. Arrowhead = protected band. Panel b is a gel that confirms α-KG binding specifically to ATP5B using DARTS and Western blotting. Panel c is a graph showing the inhibition of ATP synthase by α-KG (released from octyl α-KG). This inhibition was reversible (not shown). Panels d-g show reduced ATP levels in (Panel d) octyl α-KG treated normal human fibroblasts (***P* = 0.0016, *****P* < 0.0001) and (Panel e) α-KG treated worms (day 2, *P* = 0.969; day 8, **P* = 0.012). Decreased oxygen consumption rates are shown in Panel f for octyl α-KG treated cells (****P* = 0.0004, *****P* < 0.0001) and Panel g for α-KG treated worms (*P* < 0.0001). RLU, relative luminescence unit. Panel h shows α-KG, released from octyl α-KG (800 µM), decreases state 3, but not state 4o or 3u (*P* = 0.997), respiration in mitochondria isolated from mouse liver. The respiratory control ratio is decreased in the octyl α-KG (3.1 ± 0.6) vs. vehicle (5.2 ± 1.0) (**P* = 0.015). Panel i is an Eadie-Hofstee plot of steady-state inhibition kinetics of ATP synthase by α-KG (produced by *in situ* hydrolysis of octyl α-KG). [*S*] is the substrate (ADP) concentration, and *V* is the initial velocity of ATP synthesis in the presence of 200 µM octanol (vehicle control) or octyl α-KG. α-KG (produced from octyl α-KG) decreases the apparent *V*ₘₐₓ (53.9 to 26.7) and *K*ₘ (25.9 to 15.4), by nonlinear regression least squares fit. Number of independent experiments, Panels c-i: 2. Mean ± s.d. is plotted. By t-test, two-tailed, two-sample unequal variance. For Panel e, the first bar in each set is the vehicle.

As shown in Figure 3, α-KG longevity is mediated through ATP synthase and the DR/TOR axis. The effect of α-KG on the lifespan is shown for: *atp-2(RNAi)* (Panel a), *daf-2(e1370)* (Panel b), *eat-2(ad1116)* (Panel c), *CeTOR(RNAi)* (Panel d), *daf-16(mu86)* (Panel e), *pha-4(zu225)* (Panel f), or *hif-1(ia4)* (Panel g) worms. The number of independent experiments: RNAi control (6), *atp-2* (2), *CeTOR* (3), *N2* (5), *daf-2* (2), *eat-2* (2), *pha-4* (2), *daf-16* (2), *hif-1 (5).*

As shown in Figure 4, inhibition of ATP synthase by α-KG causes conserved decrease in TOR pathway activity. Panel a shows decreased phosphorylation of mTOR substrates in U87 cells treated with octyl α-KG or oligomycin. Similar results were obtained in HEK-293, normal human fibroblasts, and MEFs (not shown). Panel b shows increased autophagy in animals treated with α-KG or RNAi for *atp-2* or *CeTOR.* Panel c shows GFP::LGG-1 puncta quantitated using ImageJ. 2-3 independent experiments. Bars indicate the mean. *****P* < 0.0001; NS, not significant. Panel d shows that α-KG levels are increased in starved worms (***P* < 0.01). Mean ± s.d. is plotted. By *t*-test, two-tailed, two-sample unequal variance in Panels c-d. Panel e is a schematic model of α-KG-mediated longevity. As α-KG levels are elevated in starved *C*. *elegans* (Panel d), α-KG may mediate lifespan extension by a mechanism that is similar to the starvation/DR pathway (Panel e).

As shown in Figure 5, supplementation with α-KG extends *C*. *elegans* adult lifespan but does not change the growth rate of bacteria, or food intake, pharyngeal pumping rate or brood size of the worms. Panel a shows robust lifespan extension in adult *C*. *elegans* by α-KG. 8 mM α-KG increased the mean lifespan of N2 by an average of 47.3% in three independent experiments (*P* < 0.0001 for every experiment, by log-rank test). Expt. #1, *m*ᵥₑₕ = 18.9 *(n* = 87), *m*_{α-KG} = 25.8 *(n* = 96); Expt. #2, *m*ᵥₑₕ = 17.5 *(n =* 119), *m*_{α-KG} = 25.4 *(n* = 97); Expt. #3, *m*ᵥₑₕ = 16.3 *(n* = 100), *m*_{α-KG} = 26.1 *(n =* 104). *m*, mean lifespan (days of adulthood); *n,* number of animals tested. Panel b shows worms supplemented with 8 mM α-KG and worms with RNAi knockdown of α-KGDH (encoded by *ogdh-1*) have increased α-KG levels. Young adult worms were placed on treatment plates seeded with control HT115 *E. coli* or HT115 expressing *ogdh-1* dsRNA, and α-KG content was assayed after 24 hours. Panel c shows that α-KG treatment beginning at the egg stage and that beginning in adulthood produced identical lifespan increases. Vehicle, vehicle, treatment with vehicle control throughout larval and adult stages (*m* = 15.6, *n* = 95); Vehicle, α-KG, treatment with vehicle during larval stages and with 8 mM α-KG at adulthood (*m* = 26.3, *n* = 102), *P* < 0.0001 (log-rank test); α-KG, α-KG, treatment with 8 mM α-KG throughout larval and adult stages (*m* = 26.3, *n* = 102), *P* < 0.0001 (log-rank test). *m*, mean lifespan (days of adulthood); *n,* number of animals tested. Panel d shows that α-KG does not alter the growth rate of the OP50 *E. coli,* which is the standard laboratory food source for nematodes. α-KG (8 mM) or vehicle (H₂O) was added to standard LB media and the pH was adjusted to 6.6 by the addition of NaOH. Bacterial cells from the same overnight OP50 culture were added to the LB ± α-KG mixture at a 1:40 dilution, and then placed in the 37 °C incubator shaker at 300 rpm. The absorbance at 595 nm was read at 1 hour time intervals to generate the growth curve. Panel e is a schematic representation of food preference assay. Panel f is a graph showing that N2 worms show no preference between OP50 *E. coli* food treated with vehicle or α-KG (*P* = 0.85, by *t*-test, two-tailed, two-sample unequal variance), nor preference between identically treated OP50 *E. coli.* Panel g shows that the pharyngeal pumping rate of *C*. *elegans* on 8 mM α-KG is not significantly altered (by *t*-test, two-tailed, two-sample unequal variance). Panel h shows the brood size of *C*. *elegans* treated with 8 mM α-KG. Brood size analysis was conducted at 20 °C. 10 L4 wild-type worms were each singly placed onto an NGM plate containing vehicle or 8 mM α-KG. Worms were transferred one per plate onto a new plate every day, and the eggs laid were allowed to hatch and develop on the previous plate. Hatchlings were counted as a vacuum was used to remove them from the plate. Animals on 8 mM α-KG showed no significant difference in brood size compared with animals on vehicle plates (*P* = 0.223, by *t*-test, two-tailed, two-sample unequal variance). Mean ± s.d. is plotted in all cases.

As shown in Figure 6, a-KG binds to the beta subunit of ATP synthase and inhibits the activity of Complex V but not the other ETC complexes. Panel a is a Western blot showing protection of the ATP-2 protein from Pronase digestion upon α-KG binding in the DARTS assay. The antibody for human ATP5B (Sigma, AV48185) recognizes the epitope ₁₄₄IMNVIGEPIDERGPIKTKQFAPIHAEAPEFMEMSVEQEILVTGIKVVDLL₁₉₃ (SEQ ID NO:7) that has 90% identity to the *C*. *elegans* ATP-2. The lower molecular weight band near 20 kDa is a proteolytic fragment of the full-length protein corresponding to the domain directly bound by α-KG. Panel b shows that α-KG does not affect Complex IV activity. Complex IV activity was assayed using the MitoTox OXPHOS Complex IV Activity Kit (Abcam, ab109906). Relative Complex IV activity was compared to vehicle (H₂O) controls. Potassium cyanide (Sigma, 60178) was used as a positive control for the assay. Complex V activity was assayed using the MitoTox Complex V OXPHOS Activity Microplate Assay (Abcam, ab109907). Panel c shows that *atp-2(RNAi)* worms have lower oxygen consumption compared to control (*gfp* in RNAi vector), *P* < 0.0001 (*t*-test, two-tailed, two-sample unequal variance) for the entire time series (2 independent experiments); similar to α-KG treated worms shown in Figure 2, Panel g. Panel d shows that α-KG does not affect the electron flow through the electron transport chain. OCR from isolated mouse liver mitochondria at basal (pyruvate and malate as Complex I substrate and Complex II inhibitor, respectively, in presence of FCCP) and in response to sequential injection of rotenone (Rote; Complex I inhibitor), succinate (Succ; Complex II substrate), antimycin A (AA; Complex III inhibitor), ascorbate / tetramethylphenylenediamine (Asc/TMPD; cytochrome c (Complex IV) substrate). No difference in Complex I (C I), Complex II (C II), or Complex IV (C IV) respiration was observed after 30 minutes treatment with 800 µM of octyl α-KG, whereas Complex V was inhibited (see Figure 2, Panel h) by the same treatment (2 independent experiments). Panels e-f show no significant difference in coupling (Panel e) or electron flow (Panel f) was observed with either octanol or DMSO vehicle control. Panels g-h show that treatment with 1-octyl α-KG or 5-octyl α-KG gave identical results in coupling (Panel g) or electron flow (Panel h) assays. Mean ± s.d. is plotted in all cases.

As shown in Figure 7, treatment with oligomycin extends *C*. *elegans* lifespan and enhances autophagy in a manner dependent on *let-363.* Panel a shows that oligomycin extends the lifespan of adult *C*. *elegans* in a concentration dependent manner. Treatment with oligomycin began at the young adult stage. 40 µM oligomycin increased the mean lifespan of N2 worms by 32.3% (*P* < 0.0001, by log-rank test); see Figure 13 for details. Panel b shows confocal images of GFP::LGG-1 puncta in L3 epidermis of *C*. *elegans* with vehicle, oligomycin (40 µM), or α-KG (8 mM), and number of GFP::LGG-1 containing puncta quantitated using ImageJ. Bars indicate the mean. Autophagy in *C*. *elegans* treated with oligomycin or α-KG is significantly higher than in vehicle-treated control animals (*t*-test, two-tailed, two-sample unequal variance). Panel c shows there is no significant difference (n.s.) between control worms treated with oligomycin and *CeTOR(RNAi)* worms treated with vehicle, nor between vehicle and α-KG treated *CeTOR(RNAi)* worms, consistent with independent experiments in Figure 4, Panels b-c; also, oligomycin does not augment autophagy in *CeTOR(RNAi)* worms (if anything, there may be a small decrease*); by *t*-test, two-tailed, two-sample unequal variance. Bars indicate the mean.

Figure 8 shows analyses of oxidative stress in worms treated with a-KG or *atp-2* RNAi. Panel a shows that the *atp-2(RNAi)* worms have higher levels of DCF fluorescence than *gfp* control worms (*P* < 0.0001, by *t*-test, two-tailed, two-sample unequal variance). Supplementation with α-KG also leads to higher DCF fluorescence, in both HT115 (for RNAi) and OP50 fed worms (*P* = 0.0007, and *P* = 0.0012, respectively). ROS levels were measured using 2',7'-dichlorodihydrofluorescein diacetate (H₂DCF-DA). Since whole worm lysates were used, total cellular oxidative stress was measured here. H₂DCF-DA (Molecular Probes, D399) was dissolved in ethanol to a stock concentration of 1.5 mg/ml. Fresh stock was prepared every time prior to use. For measuring ROS in worm lysates, a working concentration of H₂DCF-DA at 30 ng/ml was hydrolyzed by 0.1 M NaOH at room temperature for 30 minutes to generate 2',7'-dichlorodihydrofluorescein (DCFH) before mixing with whole worm lysates in a black 96-well plate (Greiner Bio-One). Oxidation of DCFH by ROS yields the highly fluorescent 2',7'-dichlorofluorescein (DCF). DCF fluorescence was read at excitation / emission of 485 / 530 nm using SpectraMax MS (Molecular Devices). H₂O₂ was used as positive control (not shown). To prepare the worm lysates, synchronized young adult animals were cultivated on plates containing vehicle or 8 mM α-KG and OP50 or HT115 *E. coli* for 1 day, and then collected and lysed as described herein. Mean ± s.d. is plotted. Panel b shows that there was no significant change in protein oxidation upon α-KG treatment or *atp-2(RNAi).* Oxidized protein levels were determined by the OxyBlot. Synchronized young adult N2 animals were placed onto plates containing vehicle or 8 mM α-KG, and seeded with OP50 or HT115 bacteria that expressed control or *atp-2* dsRNA. Adult day 2 and day 3 worms were collected and washed 4 times with M9 buffer, and then stored at -80 °C for at least 24 hours. Laemmli buffer (Biorad, 161-0737) was added to every sample and animals were lysed by alternate boil/freeze cycles. Lysed animals were centrifuged at 14,000 rpm for 10 minutes at 4 °C to pellet worm debris, and supernatant was collected for oxyblot analysis. Protein concentration of samples was determined by the 660 nm Protein Assay (Thermo Scientific, 1861426) and normalized for all samples. Carbonylation of proteins in each sample was detected using the OxyBlot Protein Oxidation Detection Kit (Millipore, S7150).

Figure 9 shows lifespans of α-KG in the absence of *aak-2, daf-16, hif-1, vhl-1* or *egl-9.* Panel a shows N2 worms, *m*ᵥₑₕ = 17.5 *(n* = 119), *m*_{α-KG} = 25.4 *(n* = 97), *P <* 0.0001; or *aak-2(ok524)* mutants, *m*ᵥₑₕ = 13.7 *(n* = 85), *m*_{α-KG} = 17.1 *(n* = 83), *P <* 0.0001. Panel b shows N2 worms fed *gfp* RNAi control, *m*ᵥₑₕ = 18.5 *(n* = 101), *m*_{α-KG} = 23.1 (*n* = 98), *P* < 0.0001; or *daf-16* RNAi, *m*ᵥₑₕ = 14.3 *(n* = 99), *m*_{α-KG} = 17.6 *(n* = 99), *P* < 0.0001. Panel c shows N2 worms, *m*ᵥₑₕ = 21.5 *(n =* 101), *m*_{α-KG} = 24.6 (*n =* 102), *P <* 0.0001; *hif-1(ia7)* mutants, *m*ᵥₑₕ = 19.6 *(n =* 102), *m*_{α-KG} = 23.6 *(n =* 101), *P* < 0.0001; *vhl-1(ok161)* mutants, *m*ᵥₑₕ = 20.0 *(n* = 98), *m*_{α-KG} = 24.9 *(n =* 100), *P* < 0.0001; or *egl-9(sa307)* mutants, *m*ᵥₑₕ = 16.2 *(n* = 97), *m*_{α-KG} = 25.6 *(n* = 96), *P* < 0.0001. m, mean lifespan (days of adulthood); *n*, number of animals tested. P-values were determined by the log-rank test. Number of independent experiments: *N2* (8), *hif-1* (5), *vhl-1* (1), and *egl-9* (2); see Figure 13 for details. Two different *hif-1* mutant alleles (Zhang, et al. PLoS One 4, e6348 (2009)) have been used: *ia4* (shown in Figure 3, Panel g) is a deletion over several introns and exons; *ia7* (shown above) is an early stop codon, causing a truncated protein. Both alleles have the same effect on lifespan. Both alleles were tested for α-KG longevity and obtained the same results.

As shown in Figure 10, α-KG decreases TOR pathway activity but does not directly interact with TOR. Panel a shows that phosphorylation of S6K (T389) was decreased in U87 cells treated with octyl α-KG, but not in cells treated with octanol control. Same results were obtained using HEK-293 and MEF cells. Panel b shows phosphorylation of AMPK (T172) is upregulated in WI-38 cells upon Complex V inhibition by α-KG, consistent with decreased ATP content in α-KG treated cells and animals. However, this activation of AMPK appears to involve more severe Complex V inhibition than the inactivation of mTOR, as either oligomycin or a higher concentration of octyl α-KG increased P-AMPK whereas concentrations of octyl α-KG comparable to those that decreased cellular ATP content (Figure 2, Panel d) or oxygen consumption (Figure 2, Panel f) were also sufficient for decreasing P-S6K. Same results were obtained using U87 cells. Western blotting was performed with specific antibodies against P-AMPK T172 (Cell Signaling Technology, 2535S) and AMPK (Cell Signaling Technology, 2603S). Panel c shows that α-KG still induces autophagy in *aak-2(RNAi)* worms; ***P* < 0.01 (*t*-test, two-tailed, two-sample unequal variance). Number of GFP::LGG-1 containing puncta was quantitated using ImageJ. Bars indicate the mean. Panels d-e show that α-KG does not bind to TOR directly as determined by DARTS. HEK-293 (Panel d) or HeLa (Panel e) cells were lysed in M-PER buffer (Thermo Scientific, 78501) with the addition of protease inhibitors (Roche, 11836153001) and phosphatase inhibitors (50 mM NaF, 10 mM β-glycerophosphate, 5 mM sodium pyrophosphate, 2 mM Na₃VO₄). Protein concentration of the lysate was measured by BCA Protein Assay kit (Pierce, 23227). Chilled TNC buffer (50 mM Tris-HCl pH 8.0, 50 mM NaCl, 10 mM CaCl₂) was added to the protein lysate, and the protein lysate was then incubated with vehicle control (DMSO) or varying concentrations of α-KG for 1 hour (for Panel d) or 3 hours (for Panel e) at room temperature. Pronase (Roche, 10165921001) digestions were performed for 20 minutes at room temperature, and stopped by adding SDS loading buffer and immediately heating at 95 °C for 5 minutes (for Panel d) or 70 °C for 10 minutes (for Panel e). Samples were subjected to SDS-PAGE on 4-12% Bis-Tris gradient gel (Invitrogen, NP0322BOX) and Western blotted with specific antibodies against ATP5B (Santa Cruz, sc58618), mTOR (Cell Signaling Technology, 2972), or GAPDH (Ambion, AM4300). ImageJ was used to quantify the mTOR/GAPDH and ATP5B/GAPDH ratios. Susceptibility of the mTOR protein to Pronase digestion is unchanged in the presence of α-KG, whereas, as expected, Pronase resistance in the presence of α-KG is increased for ATP5B. Panel f shows increased autophagy in HEK-293 cells treated with octyl α-KG was confirmed by Western blot analysis of MAP1 LC3 (Novus, NB100-2220), consistent with decreased phosphorylation of the autophagy initiating kinase ULK1 (Figure 4, Panel a).

As shown in Figure 11, autophagy is enhanced in *C*. *elegans* treated with *ogdh-1* RNAi. Panel a shows confocal images of GFP::LGG-1 puncta in the epidermis of mid L3 stage, control or *ogdh-1* knockdown, *C*. *elegans* treated with vehicle or α-KG (8 mM). Panel b shows the number of GFP::LGG-1 puncta quantitated using ImageJ. Bars indicate the mean. *ogdh-1 (RNAi)* worms have significantly higher autophagy levels, and α-KG does not significantly augment autophagy in *ogdh-1(RNAi)* worms (*t-*test, two-tailed, two-sample unequal variance).

As shown in Figure 14, 2-HG extends the lifespan of adult C. *elegans.* Panel A shows the chemical structures of 2-hydroxyglutaric acid and α-ketoglutaric acid. Panel B shows the lifespan of (*R*)-2-HG supplemented worms is similar to worms supplemented with α-KG. Mean lifespan (days of adulthood) with vehicle treatment *m*ᵥₑₕ = 14.0 *(n =* 112 animals tested); *m*_{α-KG} = 20.7 (*n* = 114), *P* < 0.0001 (log-rank test); *m*_{(*R*)-2-HG} = 20.0 *(n =* 110), *P* < 0.0001 (log-rank test); *m*_{Met} = 14.7 *(n* = 116), *P* = 0.4305 (log-rank test); *m*_{Leu} = 13.2 *(n* = 110), *P* = 0.3307 (log-rank test). Panel C shows the lifespan of (S)-2-HG supplemented worms is similar to worms supplemented with α-KG. *m*ᵥₑₕ = 15.7 *(n* = 85); *m*_{Na2α-KG} = 21.5 *(n* = 99), *P* < 0.0001 (log-rank test); *m*_{(*S*)-2-HG} = 20.7 (*n* = 87), *P <* 0.0001 (log-rank test). All metabolites were given at a concentration of 8 mM.

As shown in Figure 15, 2-HG binds and inhibits ATP synthase. Panel A is a gel showing that ATP5B is a 2-HG binding protein as identified by DARTS and Western blotting. Panel B shows inhibition of ATP synthase by 2-HG. 2-HG, released from octyl 2-HG (600 µM), decreases state 3 (initiated by 2 mM ADP), but not state 4o (oligomycin insensitive, that is, Complex V independent) or 3u (FCCP-uncoupled maximal respiratory capacity), respiration in mitochondria isolated from mouse liver. Octanol is used as vehicle. Oligo, oligomycin; FCCP, carbonyl cyanide-*4-*(trifluoromethoxy)phenylhydrazone; AA, antimycin A. Panel C shows decreased ATP content in U87 cells treated with octyl 2-HG or octyl α-KG (**P* < 0.05, ****P* < 0.001; unpaired t-test, two-tailed, two-sample unequal variance). Octanol has no effect on ATP content. Panel D shows decreased respiration as indicated by OCR (***P* < 0.01, unpaired *t*-test, two-tailed, two-sample unequal variance) in octyl 2-HG treated U87 cells in glucose media. Octanol shows no effect on OCR compared to DMSO. Mean ± s.d. is plotted.

Figure 16 shows inhibition of ATP synthase in IDH1(R132H) cells. Panel A shows decreased ATP levels in U87 IDH1(R132H) cells (***P* = 0.0071). By unpaired *t-*test, two-tailed, two-sample unequal variance. Mean ± s.d. is plotted in all cases. Panel B) Decreased respiration in U87 IDH1(R132H) cells (***P* = 0.0037). Panel C shows 2-HG accumulation in U87/IDH1(R132H) cells *(***P* = 0.0003). Panel D shows the metabolic profile of octyl *(R)*-2-HG treated U87 cells (***P < 0.001, **P* = 0.0435). Panel E is a schematic model of metabolite signaling by α-KG and 2-HG through ATP synthase inhibition.

Figures 17A-I show metabolic vulnerability in cells with ATP5B knockdown, 2-HG accumulation, or IDH mutations. Figure 17A shows that U87/IDH1(R132H) cells have increased sensitivity to glucose starvation (***P < 0.001). Figures 17B-D show that octyl α-KG or octyl 2-HG treated U87 cells exhibit decreased viability upon glucose starvation (****P < 0.0001, ***P < 0.001, **P < 0.01, *P < 0.05). Octanol has no effect on viability. Figure 17E) ATP5B knockdown inhibits U87 cell growth (***P = 0.0004). Figure 17F shows that HCT 116 IDH1(R132H/+) cells exhibit increased vulnerability to glucose-free medium supplemented with (R)-3-hydroxybutyrate (***P < 0.001). By unpaired t-test, two-tailed, two-sample unequal variance. Mean ± s.d. is plotted in all cases. Figures 17G-I show that U87 cells with ATP5B knockdown, membrane-permeable esterase-hydrolysable analogs of α-KG or 2-HG treatment, or stably expressing IDH1(R132H) exhibit decreased mTOR Complex 1 activity in glucose-free, galactose-containing medium. S6K (T389) and 4E-BP1 (S65) are substrates of mTOR Complex 1. Octanol exhibits no effect on TOR activity.

As shown in Figure 18, 2-HG does not affect the electron flow through the electron transport chain and does not affect ADP import. Panel A shows OCR from isolated mouse liver mitochondria at basal (pyruvate and malate as Complex I substrate and Complex II inhibitor, respectively, in presence of FCCP) and in response to sequential injection of rotenone (Rote; Complex I inhibitor), succinate (Complex II substrate), antimycin A (AA; Complex III inhibitor), tetramethylphenylenediamine (TMPD; cytochrome c (Complex IV) substrate). No difference in Complex I (C I), Complex II (C II), or Complex IV (C IV) respiration is observed after 30 minute treatment with 600 µM of octyl 2-HG, whereas Complex V is inhibited (Figure 15, Panel B) by the same treatment (2 independent experiments). Octanol is used as vehicle. Panel B shows ADP import was measured in the presence of octanol (vehicle control) or octyl 2-HG (600 µM). Octyl *(R)*-2-HG, *P* = 0.4237; octyl *(S)*-2-HG, *P* = 0.1623). CATR (carboxyatractyloside), a known inhibitor for ADP import, was used as a positive control for the assay (****P* = 0.0003). By unpaired t-test, two-tailed, two-sample unequal variance. Mean ± s.d. is plotted in all cases.

As shown in Figure 19, 2-HG inhibits cellular respiration and decreases ATP levels. Panel A shows oligomycin, a known inhibitor of ATP synthase, is used as a positive control (**P* < 0.05; unpaired t-test, two-tailed, two-sample unequal variance). Panels B-C show U87 cells treated with octyl 2-HG have decreased ATP synthase dependent (oligomycin sensitive) oxygen consumption rate (OCR) (**P* < 0.05, ***P <* 0.01, ****P* < 0.001; unpaired t-test, two-tailed, two-sample unequal variance). Mean ± s.d. is plotted in all cases.

Figure 20 shows cellular energetics and metabolic profiles of 2-HG accumulated cells. Panel A shows HCT 116 IDH1(R132H/+) cells exhibit decreased respiration (***P* = 0.0015). Panel B shows 2-HG levels are about 100 folder higher in HCT 116/IDH1 (R132H/+) cells than in parental control cells (*****P* < 0.0001). Panel C shows the metabolic profile of TCA cycle intermediates and related amino acids in octyl *(S)*-2-HG treated U87 cells (**P* < 0.05). Panel D shows the metabolic profile of TCA cycle intermediates and related amino acids in octyl α-KG treated U87 cells (****P* < 0.001, ***P* < 0.01). Unpaired t-test, two-tailed, two-sample unequal variance. Mean ± s.d. is plotted in all cases.

As shown in Figure 21, HCT 116 IDH1(R132H/+) cells exihibit metabolic vulnerability and growth inhibition. Panel A shows that HCT 116 IDH1(R132H/+) cells have increased sensitivity to glucose starvation. Panel B shows that HCT 116 IDH1(R132H/+) cells present decreased growth rate. ***P* < 0.01; unpaired t-test, two-tailed, two-sample unequal variance. Mean ± s.d. is plotted in all cases.

Figure 22 shows cell growth inhibition upon ATP5B knockdown, treatment with octyl 2-HG or octyl α-KG, or IDH1(R132H) mutation. Panel A shows that even in glucose medium, ATP5B knockdown decreases the growth rate of U87 cells. Panels B-D show that U87 cells exhibit decreased growth rate upon octyl α-KG or octyl 2-HG treatment in glucose-free medium. Growth rate was reduced also in glucose medium albeit to a lesser extent (not shown). Panel E shows that U87 cells expressing IDH1(R132H) exhibit decreased proliferation rate even in glucose medium. *****P* < 0.0001, ****P* < 0.001, ***P* < 0.01, **P* < 0.05; unpaired t-test, two-tailed, two-sample unequal variance. Mean ± s.d. is plotted in all cases.

In experiments similar to those conducted for a-KG and 2-HG compounds, both D-glutamate and L-glutamate administration increases the lifespan of subjects (data not shown).

Having thus described exemplary embodiments of the present invention, it should be noted by those skilled in the art that the within disclosures are exemplary only and that various other alternatives, adaptations, and modifications may be made within the scope of the present invention. Accordingly, the present invention is not limited to the specific embodiments as illustrated herein, but is only limited by the following claims.

## Claims

1. A compound for use in treating cardiac hypertrophy in a subject in need thereof, said compound selected from 1-octyl alpha-ketoglutarate and pharmaceutically acceptable solvates and salts thereof, wherein the heart size is reduced and the cardiac output of the subject is restored to pre-cardiac hypertrophic output levels.

2. A compound for use in treating cardiac hypertrophy in a subject in need thereof, said compound selected from 5-octyl alpha-ketoglutarate and pharmaceutically acceptable solvates and salts thereof, wherein the heart size is reduced and the cardiac output of the subject is restored to pre-cardiac hypertrophic output levels.

3. A compound for use in treating cardiac hypertrophy in a subject in need thereof, said compound selected from 2-hydroxyglutaric acid, 2-hydroxypentanedioate, 1-octyl-(S)-2-hydroxypentanedioate, 1-octyl-(R)-2-hydroxypentanedioate, 5-octyl-(S)-2-hydroxypentanedioate, 5-octyl-(R)-2-hydroxypentanedioate, and pharmaceutically acceptable solvates and salts thereof, wherein the heart size is reduced and the cardiac output of the subject is restored to pre-cardiac hypertrophic output levels.

4. The compound for use according to any one of claims 1 to 3, wherein the compound is administered as a daily dose of about 0.25-2 grams per kilogram weight of the subject per day.

5. The compound for use according to any one of claims 1 to 3, wherein the compound is administered as a daily dose of about 0.5-2 grams per kilogram weight of the subject per day.

6. The compound for use according to any one of claims 1 to 3, wherein the compound is administered as a daily dose of about 1-2 grams per kilogram weight of the subject per day.

7. The compound for use according to any one of claims 1 to 3, wherein the compound is administered as a daily dose of about 2 grams per kilogram weight of the subject per day.

## Patentansprüche

1. Verbindung zur Verwendung bei einem Behandeln von Herzhypertrophie bei einem Subjekt, der dies benötigt, wobei die Verbindung ausgewählt ist aus 1-Octylalpha-ketoglutarat und pharmazeutisch verträglichen Solvaten und Salzen davon, wobei die Herzgröße reduziert ist und die Herzleistung des Subjekts auf das Leistungsniveau vor der Herzhypertrophie zurückgeführt wird.

2. Verbindung zur Verwendung bei einem Behandeln von Herzhypertrophie bei einem Subjekt, der dies benötigt, wobei die Verbindung ausgewählt ist aus 5-Octylalpha-ketoglutarat und pharmazeutisch verträglichen Solvaten und Salzen davon, wobei die Herzgröße reduziert ist und die Herzleistung des Subjekts auf das Leistungsniveau vor der Herzhypertrophie zurückgeführt wird.

3. Verbindung zur Verwendung bei einem Behandeln von Herzhypertrophie bei einem Subjekt, der dies benötigt, wobei die Verbindung ausgewählt ist aus 2-Hydroxyglutarsäure, 2-Hydroxypentandioat, 1-Octyl-(S)-2-hydroxypentandioat, 1-Octyl-(R)-2-hydroxypentandioat, 5-Octyl-(S)-2-hydroxypentandioat, 5-Octyl-(R)-2-hydroxypentandioat und pharmazeutisch verträglichen Solvaten und Salzen davon, wobei die Herzgröße reduziert ist und die Herzleistung des Subjekts auf das Leistungsniveau vor der Herzhypertrophie zurückgeführt wird.

4. Verbindung zur Verwendung nach einem der Ansprüche 1 bis 3, wobei die Verbindung als eine Tagesdosis von etwa 0,25-2 Gramm pro Kilogramm Gewicht des Subjekts pro Tag verabreicht wird.

5. Verbindung zur Verwendung nach einem der Ansprüche 1 bis 3, wobei die Verbindung als eine Tagesdosis von etwa 0,5-2 Gramm pro Kilogramm Gewicht des Subjekts pro Tag verabreicht wird.

6. Verbindung zur Verwendung nach einem der Ansprüche 1 bis 3, wobei die Verbindung als eine Tagesdosis von etwa 1-2 Gramm pro Kilogramm Gewicht des Subjekts pro Tag verabreicht wird.

7. Verbindung zur Verwendung nach einem der Ansprüche 1 bis 3, wobei die Verbindung als eine Tagesdosis von etwa 2 Gramm pro Kilogramm Körpergewicht des Patienten pro Tag verabreicht wird.

## Revendications

1. Composé pour une utilisation dans le traitement d'une hypertrophie cardiaque chez un sujet qui en a besoin, ledit composé étant choisi parmi 1-octyl alpha-cétoglutarate et solvants et sels pharmaceutiquement acceptables de celui-ci, dans lequel la taille du coeur est réduite et le débit cardiaque du sujet est rétabli à des niveaux de débit précardiaque hypertrophique.

2. Composé pour une utilisation dans le traitement d'une hypertrophie cardiaque chez un sujet qui en a besoin, ledit composé étant choisi parmi 5-octyl alpha-cétoglutarate et solvants et sels pharmaceutiquement acceptables de celui-ci, dans lequel la taille du coeur est réduite et le débit cardiaque du sujet est rétabli à des niveaux de débit précardiaque hypertrophique.

3. Composé pour une utilisation dans le traitement d'une hypertrophie cardiaque chez un sujet qui en a besoin, ledit composé étant choisi parmi acide 2-hydroxy glutarique, 2-hydroxypentanedioate, 1-octyl-(S)-2-hydroxypentanedioate, 1-octyl-(R)-2-hydroxypentanedioate, 5-octyl-(S)-2-hydroxypentanedioate, 5-octyl-(R)-2-hydroxypentanedioate et solvants et sels pharmaceutiquement acceptables de ceux-ci, dans lesquels la taille du coeur est réduite et le débit cardiaque du sujet est rétabli à des niveaux de débit précardiaque hypertrophique.

4. Composé pour une utilisation selon l'une quelconque des revendications 1 à 3, dans lequel le composé est administré sous la forme d'une dose quotidienne d'environ 0,25 à 2 grammes par kilogramme de poids du sujet par jour.

5. Composé pour une utilisation selon l'une quelconque des revendications 1 à 3, dans lequel le composé est administré sous la forme d'une dose quotidienne d'environ 0,5 à 2 grammes par kilogramme de poids du sujet par jour.

6. Composé pour une utilisation selon l'une quelconque des revendications 1 à 3, dans lequel le composé est administré sous la forme d'une dose quotidienne d'environ 1 à 2 grammes par kilogramme de poids du sujet par jour.

7. Composé pour une utilisation selon l'une quelconque des revendications 1 à 3, dans lequel le composé est administré sous la forme d'une dose quotidienne d'environ 2 grammes par kilogramme de poids du sujet par jour.
